# EUROPEAN PATENT APPLICATION

(11) **EP 0 844 305 A2**
(43) Date of publication of application: **27.05.1998**
(21) Application number: 97308998.0
(22) Date of filing: 10.11.1997
(51) Int. Cl.: C12N 9/90, C12Q 1/68, C07K 16/40

(54) **hRAD54**

(30) Priority: 13.11.1996 US 30676 P
(71) Applicant: SMITHKLINE BEECHAM CORPORATION, Philadelphia Pennsylvania 19103 (US); THOMAS JEFFERSON UNIVERSITY, Philadelphia Pennsylvania 19107 (US)
(72) Inventor: Croce, Carlo M., Thomas Jefferson University, Philadelphia, Pennsylvania 19107 (US); Fishel, Richard A., Thomas Jefferson University, Philadelphia, Pennsylvania 19107 (US); Rasio, Debora, Thomas Jefferson University, Philadelphia, Pennsylvania 19107 (US); Robbins, David J., SmithKline Beecham Pharm., King of Prussia, Pennsylvania 19406 (US)
(74) Representative: Connell, Anthony Christopher

(57) **Abstract**

hRAD54 polypeptides and DNA (RNA) encoding such enzyme and a procedure for producing such polypeptides by recombinant techniques is disclosed. Also disclosed are methods for utilizing such hRAD54 in the development of treatments for cancer prone syndromes, such as Xeroderma Pigmentosum and Bloom syndrome, Wemer's syndromes, and X-linked Mental Retardation with alpha-thalassemia Syndrome (ATR-X); as well as cancers, such as that of the breast, among others, are also disclosed. Also disclosed are diagnostic assays for detecting diseases related to mutations in the nucleic acid sequences and altered concentrations of the polypeptides.

## Description

### FIELD OF INVENTION

This invention relates, in part, to newly identified polynucleotides and polypeptides; variants and derivatives of the polynucleotides and polypeptides; processes for making the polynucleotides and the polypeptides, and their variants and derivatives; agonists and antagonists of the polypeptides; and uses of the polynucleotides, polypeptides, variants, derivatives, agonists and antagonists. In particular, in these and in other regards, the invention relates to novel polynucleotides and polypeptides of the family of RAD 54, hereinafter referred to as hRAD54.

### BACKGROUND OF INVENTION

A loss of DNA repair functions that result in elevated mutation rates has been proposed to explain multiple mutations found in the development of tumors. It has been shown recently that mutations of the human DNA mismatch repair genes hMSH2 and hMLH1 accounted for the majority of hereditary nonpolyposis colon cancer (HNPCC) cases. Another mechanism in which altered or defective repair functions can lead to higher mutation rates is the loss or rearrangement of chromosomes, observed in many tumors. The mechanism of these chromosomal rearrangements has been proposed to be a result of chromosome pairing disorders prior to mitotic disjunction, aberrant recombinational repair processes or loss of cell cycle check point functions that normally maintain chromosomal integrity. Studies of lower eucaryotes have demonstrated that mutations in the RAD54 gene lead to severe defects in the repair of radiation-damaged DNA as well as in spontaneous and induced mitotic recombination. A postulated role of yeast RAD54 in recombination repair is to provide access to regions of chromatin, thus making them available for recombination. A human homologue of the RAD54 gene, a DNA helicase, has been postulated. Mutations in other human DNA helicase genes have been shown to be responsible for cancer-prone syndromes such as Xeroderma Pigmentosum and Bloom syndrome, as well as Werner's syndrome (premature aging) and the X-linked Mental Retardation with a-thalassemia Syndrome (ATR-X). This suggests that a human RAD54 gene could be a candidate modifier gene in tumors that display allelic imbalance.

The gene contained in this filing, a human homologue of the RAD54 gene, maps to chromosomal region lp32. The short arm of chromosome 1 shows a complex pattern of internal deletions, suggesting the presence of tumor suppressor genes required for normal cellular maintainence. Chromosomal band lp32 has been identified as one of the four minimal regions of chromosome I deletion in breast carcinomas. Tumors of neural crest origin such as medullary thyroid carcinomas and pheochromocytomas, together with memingiomas show loss of genetic material (loss of heterozygosity, LOH) at lp32-lpter. This suggests that the hRAD54 gene described could be a candidate gene in tumors that display allelic imbalance at lp32.

### SUMMARY OF THE INVENTION

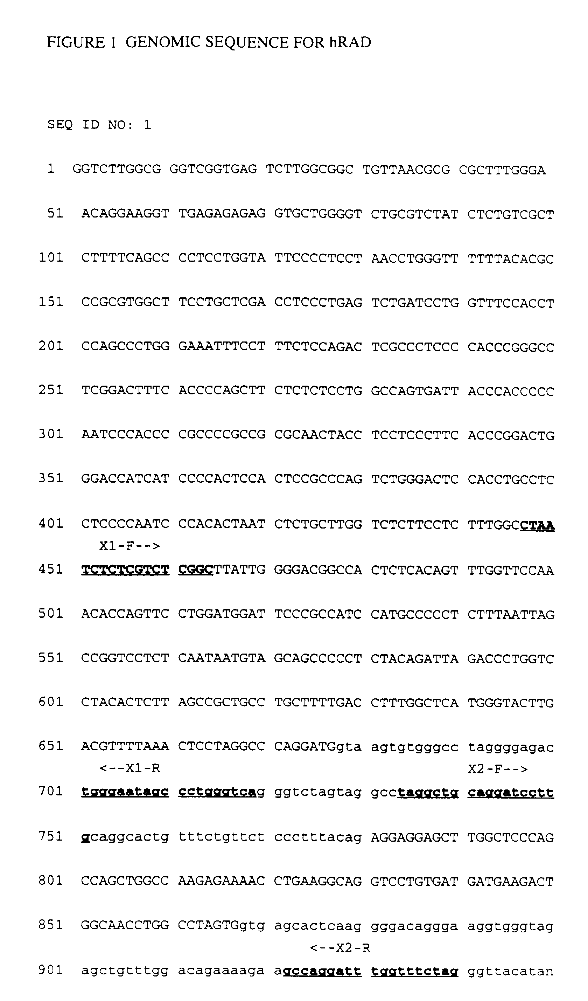

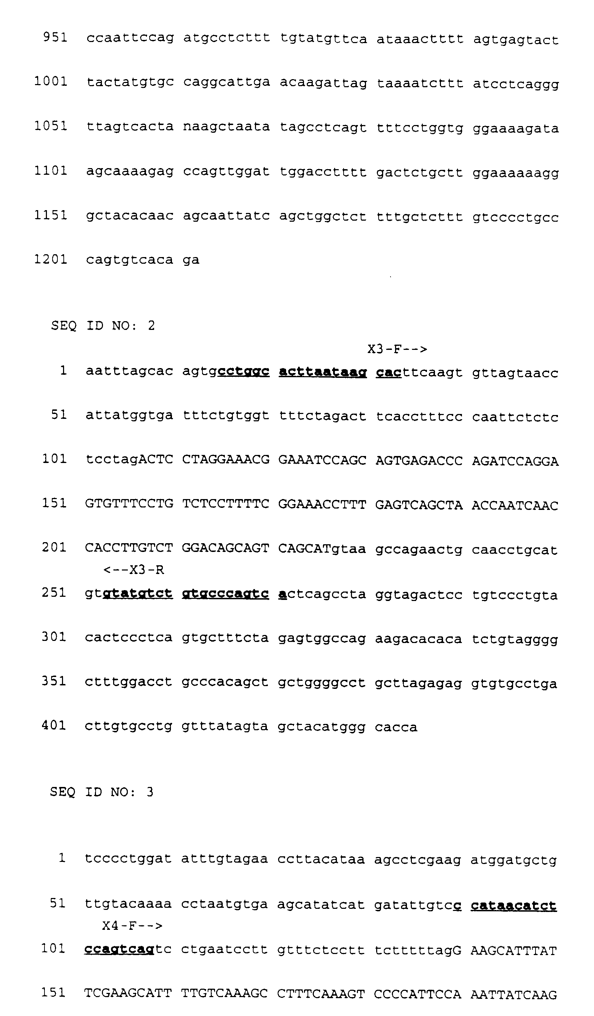

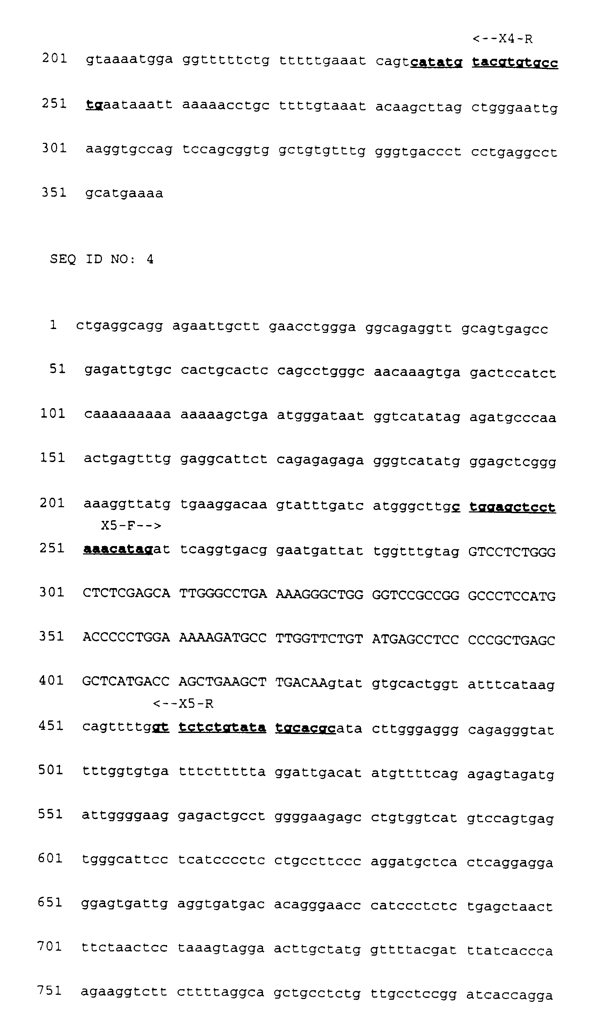

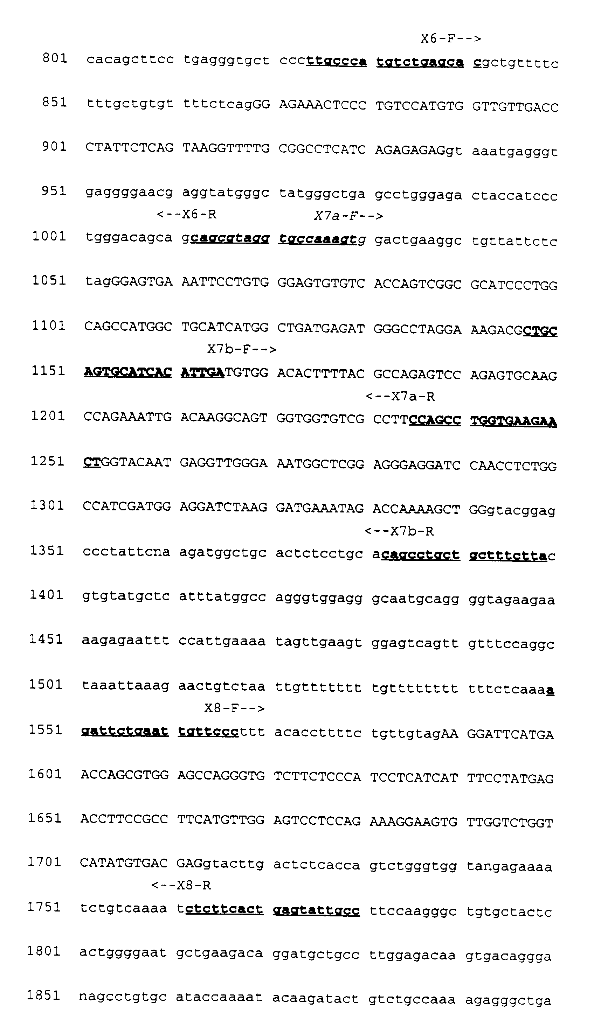

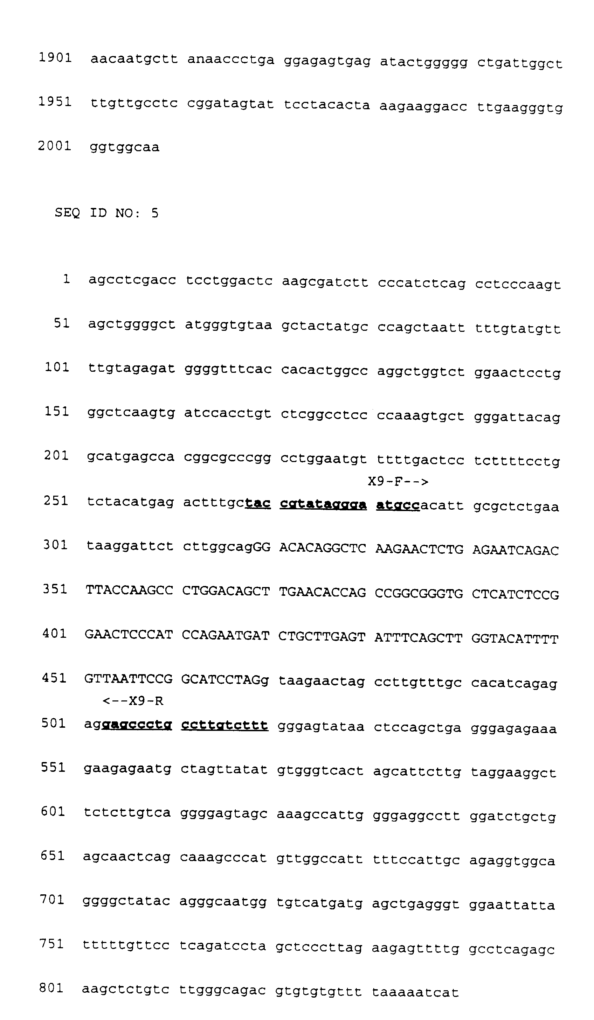

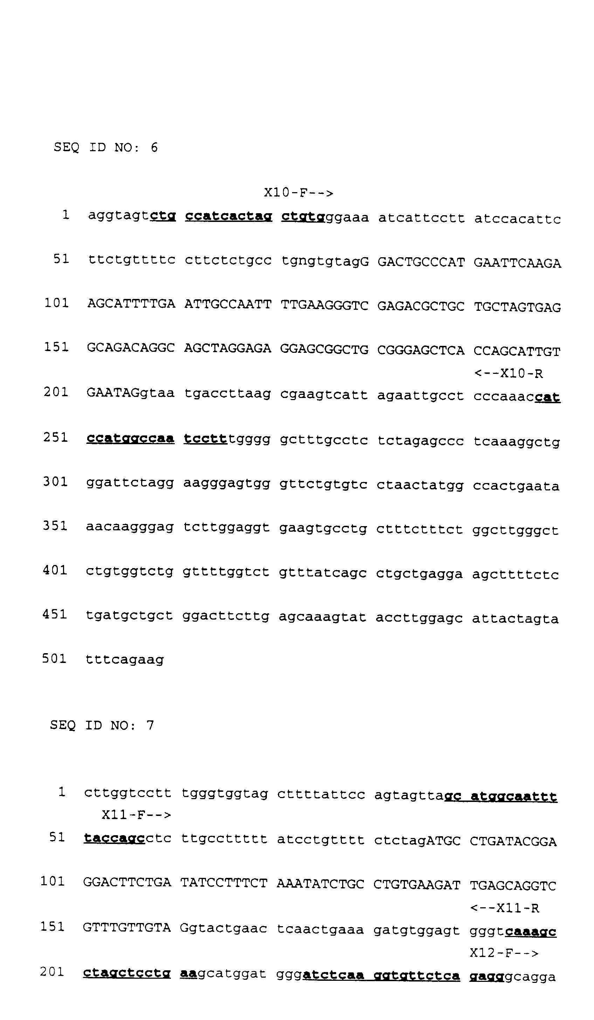

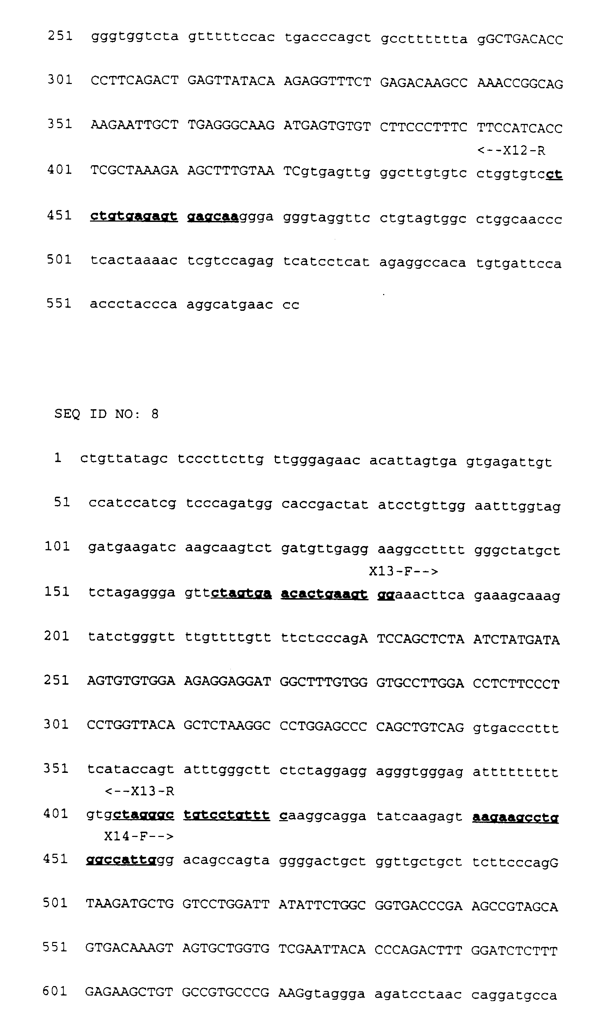

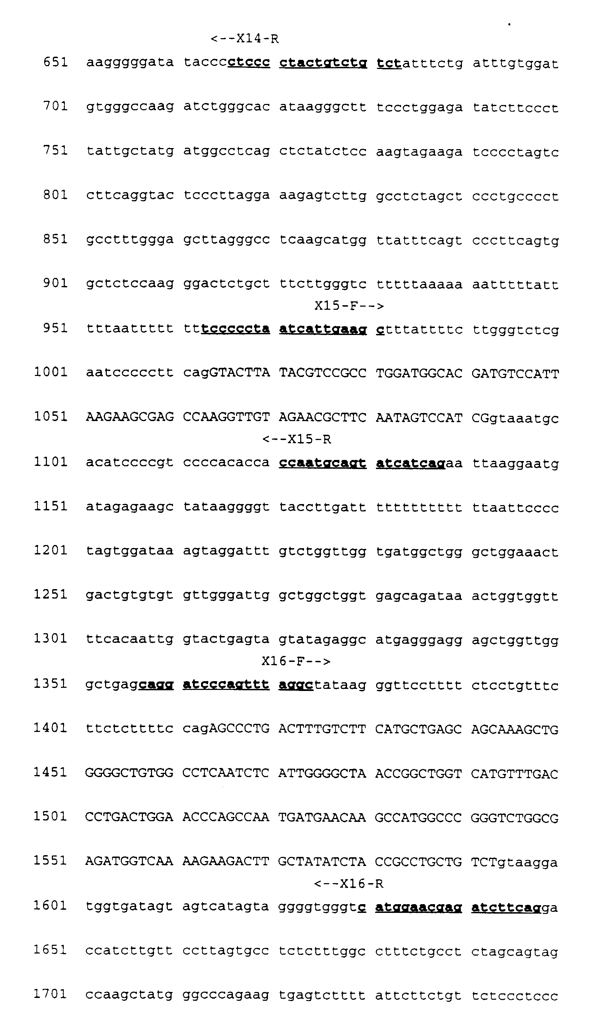

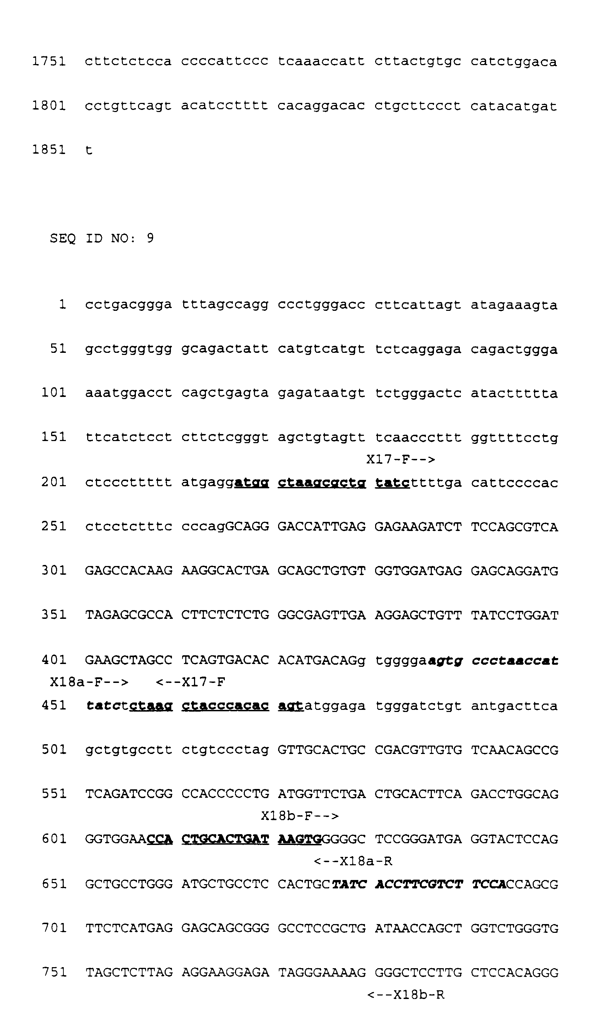

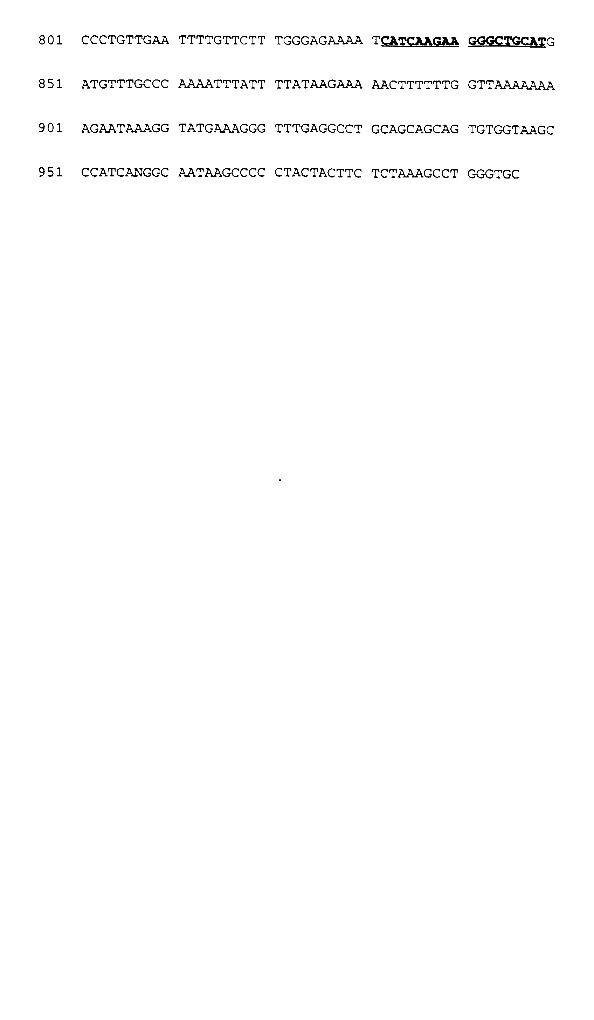

Toward these ends, and others, it is an object of the present invention to provide polypeptides, *inter alia,* that have been identified as novel hRAD54 by homology between the amino acid sequence set out in Figure 1 and known amino acid sequences of other proteins such yeast RAD54 protein.

It is a further object of the invention, moreover, to provide polynucleotides of SEQ ID NOS: 1-9. In a particularly preferred embodiment of this aspect of the invention, the polynucleotide comprises the region encoding hRAD54 in the sequence set out in SEQ ID NO: 10.

In accordance with this aspect of the invention, there are provided isolated nucleic acid molecules encoding hRAD54, including mRNAs, cDNAs, genomic DNAs and fragments and, in further embodiments of this aspect of the invention, biologically, diagnostically, clinically or therapeutically useful variants, analogs or derivatives thereof, including fragments of the variants, analogs and derivatives.

Among the particularly preferred embodiments of this aspect of the invention are naturally occurring allelic variants of hRAD54.

It also is an object of the invention to provide human polypeptides, particularly hRAD54 polypeptides, that may be employed for therapeutic purposes, for example, in the treatment of cancer prone syndromes, such as Xeroderma Pigmentosum and Bloom syndrome, Werner's syndromes, and X-linked Mental Retardation with alpha-thalassemia Syndrome (ATR-X); as well as cancers, such as that of the breast, among others.

In accordance with this aspect of the invention, there are provided novel polypeptides of human origin, referred to herein as hRAD54, as well as biologically, diagnostically or therapeutically useful fragments, variants and derivatives thereof, variants and derivatives of the fragments, and analogs of the foregoing.

Among the particularly preferred embodiments of this aspect of the invention are variants of hRAD54 encoded by naturally occurring alleles of the hRAD54 gene.

In accordance with another aspect of the present invention, there are provided methods of screening for compounds which bind to and activate or inhibit activation of the enzyme of the present invention.

It is another object of the invention to provide a process for producing the aforementioned polypeptides, polypeptide fragments, variants and derivatives, fragments of the variants and derivatives, and analogs of the foregoing. In a preferred embodiment of this aspect of the invention, there are provided methods for producing the aforementioned hRAD54 polypeptides comprising culturing host cells having expressibly incorporated therein an exogenously-derived hRAD54-encoding polynucleotide under conditions for expression of hRAD54 in the host, expressing the hRAD54 in the host cells, and then recovering the expressed polypeptide from the host cells.

In accordance with another object of the invention, there are provided products, compositions, processes and methods that utilize the aforementioned polypeptides and polynucleotides for research, biological, clinical and therapeutic purposes, *inter alia.*

In accordance with certain preferred embodiments of this aspect of the invention, there are provided products, compositions and methods, *inter alia,* for, among other things: assessing hRAD54 expression in cells by determining hRAD54 polypeptides or hRAD54-encoding mRNA; to treat cancer prone syndromes, such as Xeroderma Pigmentosum and Bloom syndrome, Wemer's syndromes, and X-linked Mental Retardation with alpha-thalassemia Syndrome (ATR-X); as well as cancers, such as that of the breast; among others, *in vitro, ex vivo* or *in vivo* by exposing cells to hRAD54 polypeptides or polynucleotides as disclosed herein; assaying genetic variation and aberrations, such as defects, in hRAD54 genes; and administering a hRAD54 polypeptide or polynucleotide to an organism to augment hRAD54 function or remediate hRAD54 dysfunction.

In accordance with still another embodiment of the present invention, there is provided a process of using such activating compounds to stimulate enzyme of the present invention for the treatment of conditions related to the under-expression of hRAD54.

In accordance with another aspect of the present invention, there is provided a process of using such inhibiting compounds for treating conditions associated with over-expression of the hRAD54.

In accordance with yet another aspect of the present invention, there is provided non-naturally occurring synthetic, isolated and/or recombinant hRAD54 polypeptides which are fragments, consensus fragments and/or sequences having conservative amino acid substitutions of at least one domain of the hRAD54 of the present invention, such polypeptide being capable of modulating, quantitatively or qualitatively, hRAD54 binding to its receptor, ligands or substrates.

In accordance with still another aspect of the present invention, there are provided synthetic or recombinant hRAD54 polypeptides, conservative substitution and derivatives thereof, antibodies thereto, anti-idiotype antibodies, compositions and methods that can be useful as potential modulators of hRAD54 function, by binding to the enzyme or modulating enzyme binding, due to their expected biological properties, which may be used in diagnostic, therapeutic and/or research applications.

It is still another object of the present invention to provide synthetic, isolated or recombinant polypeptides which are designed to inhibit or mimic various hRAD54 or fragments thereof.

In accordance with certain preferred embodiments of this and other aspects of the invention, there are provided probes that hybridize to hRAD54 sequences.

In certain additional preferred embodiments of this aspect of the invention, there are provided antibodies against hRAD54 polypeptides. In certain particularly preferred embodiments in this regard, the antibodies are highly selective for hRAD54.

In accordance with another aspect of the present invention, there are provided hRAD54 agonists. Among preferred agonists are molecules that mimic the hRAD54 enzyme, that bind to hRAD54-binding molecules or receptors, and that elicit or augment hRAD54-induced responses. Also among preferred agonists are molecules that interact with hRAD54 or hRAD54 polypeptides, or with other modulators of hRAD54 activities, thereby potentiating or augmenting an effect of hRAD54 or more than one effect of hRAD54.

In accordance with yet another aspect of the present invention, there are provided hRAD54 antagonists. Among preferred antagonists are those which mimic the hRAD54 enzyme so as to bind to hRAD54 receptors or binding molecules but not elicit a hRAD54-induced response or more than one hRAD54-induced response. Also among preferred antagonists are molecules that bind to or interact with hRAD54 so as to inhibit an effect of hRAD54 or more than one effect of hRAD54 or to prevent expression of hRAD54.

In a further aspect of the invention, there are provided compositions comprising a hRAD54 polynucleotide or a hRAD54 polypeptide for administration to cells *in vitro,* to cells *ex vivo* and to cells *in vivo,* or to a multicellular organism. In certain particularly preferred embodiments of this aspect of the invention, the compositions comprise a hRAD54 polynucleotide for expression of a hRAD54 polypeptide in a host organism for treatment of disease. Particularly preferred in this regard is expression in a human patient for treatment of a dysfunction associated with aberrant endogenous activity of hRAD54.

### BRIEF DESCRIPTION OF THE DRAWINGS

The following drawings depict certain embodiments of the invention. They are illustrative only and do not limit the invention otherwise disclosed herein.

Figure 1 shows the genomic nucleotide sequence of hRAD54 (SEQ ID NOS: 1-9). The capital letters indicate exons and small letter indicate introns. Also shown are primers for amplyfing the exons. SEQ ID NO: 1 comprises exon 1-2. SEQ ID NO: 2 comprises exon 3. SEQ ID NO: 3 comprises exon 4. SEQ ID NO 4 comprises exons 5, 6, 7, and 8. SEQ ID NO 5 comprises exon 9. SEQ ID NO 6 comprises 10. SEQ ID NO 7 comprises exon 11 and 12. SEQ ID NO 8 comprises exons 13, 14, 15 and 16. SEQ ID NO 9 comprises exons 17 and 18.

Figure 2 shows the deduced amino acid sequence of hRAD54 (SEQ ID NO: 10).

### GLOSSARY

The following illustrative explanations are provided to facilitate understanding of certain terms used frequently herein, particularly in the examples. The explanations are provided as a convenience and are not meant to limit the invention.

"Digestion" of DNA refers to catalytic cleavage of a DNA with an enzyme such as, but not limited to, a restriction enzyme that acts only at certain sequences in the DNA. The various restriction enzymes referred to herein are commercially available and their reaction conditions, cofactors and other requirements for use are known and routine to the skilled artisan.

For analytical purposes, typically, 1 microgram of plasmid or DNA fragment is digested with about 2 units of enzyme in about 20 microliters of reaction buffer. For the purpose of isolating DNA fragments for plasmid construction, typically 5 to 50 micrograms of DNA are digested with 20 to 250 units of enzyme in proportionately larger volumes.

Appropriate buffers and substrate amounts for particular restriction enzymes are described in standard laboratory manuals, such as those referenced below, and are specified by commercial suppliers.

Incubation times of about 1 hour at 37°C are ordinarily used, but conditions may vary in accordance with standard procedures, the supplier's instructions and the particulars of the reaction. After digestion, reactions may be analyzed, and fragments may be purified by electrophoresis through an agarose or polyacrylamide gel, using well known methods that are routine for those skilled in the art.

"Genetic element" generally means a polynucleotide comprising a region that encodes a polypeptide or a region that regulates replication, transcription, translation or other processes important to expression of the polypeptide in a host cell, or a polynucleotide comprising both a region that encodes a polypeptide and a region operably linked thereto that regulates expression.

Genetic elements may be comprised within a vector that replicates as an episomal element; that is, as a molecule physically independent of the host cell genome. They may be comprised within mini-chromosomes, such as those that arise during amplification of transfected DNA by methotrexate selection in eukaryotic cells. Genetic elements also may be comprised within a host cell genome, not in their natural state but, rather, following manipulation such as isolation, cloning and introduction into a host cell in the form of purified DNA or in a vector, among others.

"Isolated" means altered "by the hand of man" from its natural state; i.e., that, if it occurs in nature, it has been changed or removed from its original environment, or both. For example, a naturally occurring polynucleotide or a polypeptide naturally present in a living animal in its natural state is not "isolated," but the same polynucleotide or polypeptide separated from the coexisting materials of its natural state is "isolated", as the term is employed herein. For example, with respect to polynucleotides, the term isolated means that it is separated from the chromosome and cell in which it naturally occurs.

As part of or following isolation, such polynucleotides can be joined to other polynucleotides such as DNAs, for mutagenesis, to form fusion proteins, and for propagation or expression in a host, for instance. The isolated polynucleotides, alone or joined to other polynucleotides such as vectors, can be introduced into host cells, in culture or in whole organisms. Introduced into host cells in culture or in whole organisms, such DNAs still would be isolated, as the term is used herein, because they would not be in their naturally occurring form or environment. Similarly, the polynucleotides and polypeptides may occur in a composition, such as a media, formulations, solutions for introduction of polynucleotides or polypeptides, for example, into cells, compositions or solutions for chemical or enzymatic reactions, for instance, which are not naturally occurring compositions, and, therein remain isolated polynucleotides or polypeptides within the meaning of that term as it is employed herein.

"Ligation" refers to the process of forming phosphodiester bonds between two or more polynucleotides, which most often are double stranded DNAs. Techniques for ligation are well known to the art and protocols for ligation are described in standard laboratory manuals and references, such as, for instance, Sambrook *et al.,* MOLECULAR CLONING, A LABORATORY MANUAL, 2nd Ed.; Cold Spring Harbor Laboratory Press, Cold Spring Harbor, New York, 1989, hereinafter referred to as Sambrook *et al.*

"Oligonucleotide(s)" refers to relatively short polynucleotides. Often the term refers to single-stranded deoxyribonucleotides, but it can refer as well to single- or double-stranded ribonucleotides, RNA:DNA hybrids and double-stranded DNAs, among others.

Oligonucleotides, such as single-stranded DNA probe oligonucleotides, often are synthesized by chemical methods, such as those implemented on automated oligonucleotide synthesizers. However, oligonucleotides can be made by a variety of other methods, including *in vitro* recombinant DNA-mediated techniques and by expression of DNAs in cells and organisms.

Initially, chemically synthesized DNAs typically are obtained without a 5' phosphate. The 5' ends of such oligonucleotides are not substrates for phosphodiester bond formation by ligation reactions that employ DNA ligases typically used to form recombinant DNA molecules. Where ligation of such oligonucleotides is desired, a phosphate can be added by standard techniques, such as those that employ a kinase and ATP.

The 3' end of a chemically synthesized oligonucleotide generally has a free hydroxyl group and, in the presence of a ligase, such as T4 DNA ligase, will readily form a phosphodiester bond with a 5' phosphate of another polynucleotide, such as another oligonucleotide. As is well known, this reaction can be prevented selectively, where desired, by removing the 5' phosphates of the other polynucleotide(s) prior to ligation.

"Plasmids" are genetic elements that are stably inherited without being a part of the chromosome of their host cell. They may be comprised of DNA or RNA and may be linear or circular. Plasmids code for molecules that ensure their replication and stable inheritance during cell replication and may encode products of considerable medical, agricultural and environmental importance. For example, they code for toxins that greatly increase the virulence of pathogenic bacteria They can also encode genes that confer resistance to antibiotics. Plasmids are widely used in molecular biology as vectors used to clone and express recombinant genes. Plasmids generally are designated herein by a lower case "p" preceded and/or followed by capital letters and/or numbers, in accordance with standard naming conventions that are familiar to those of skill in the art. Starting plasmids disclosed herein are either commercially available, publicly available, or can be constructed from available plasmids by routine application of well known, published procedures. Many plasmids and other cloning and expression vectors that can be used in accordance with the present invention are well known and readily available to those of skill in the art. Moreover, those of skill may readily construct any number of other plasmids suitable for use in the invention. The properties, construction and use of such plasmids, as well as other vectors, in the present invention will be readily apparent to those of skill from the present disclosure.

"Polynucleotide(s)" generally refers to any polynbonucleotide or polydeoxribonucleotide, which may be unmodified RNA or DNA or modified RNA or DNA. Thus, for instance, polynucleotides as used herein refers to, among others, single- and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions. In addition, polynucleotide, as used herein, refers to triple-stranded regions comprising RNA or DNA or both RNA and DNA. The strands in such regions may be from the same molecule or from different molecules. The regions may include all of one or more of the molecules, but more typically involve only a region of some of the molecules. One of the molecules of a triple-helical region often is an oligonucleotide. As used herein, the term polynucleotide also includes DNAs or RNAs as described above that contain one or more modified bases. Thus, DNAs or RNAs with backbones modified for stability or for other reasons are polynucleotides, as that term is intended herein. Moreover, DNAs or RNAs comprising unusual bases, such as inosine, or modified bases, such as tritylated bases, to name just two examples, are polynucleotides, as the term is used herein. It will be appreciated that a great variety of modifications have been made to DNA and RNA that serve many useful purposes known to those of skill in the art. The term polynucleotide, as it is employed herein, embraces such chemically, enzymatically or metabolically modified forms of polynucleotides, as well as the chemical forms of DNA and RNA characteristic of viruses and cells, including *inter alia* simple and complex cells.

"Polypeptides", as used herein, includes all polypeptides as described below. The basic structure of polypeptides is well known and has been described in innumerable textbooks and other publications in the art. In this context, the term is used herein to refer to any peptide or protein comprising two or more amino acids joined to each other in a linear chain by peptide bonds. As used herein, the term refers to both short chains, which also commonly are referred to in the art as peptides, oligopeptides and oligomers, for example, and to longer chains, which generally are referred to in the art as proteins, of which there are many types.

It will be appreciated that polypeptides often contain amino acids other than the 20 amino acids commonly referred to as the 20 naturally occurring amino acids, and that many amino acids, including the terminal amino acids, may be modified in a given polypeptide, either by natural processes, such as processing and other post-translational modifications, or by chemical modification techniques which are well known to the art. Even the common modifications that occur naturally in polypeptides are too numerous to list exhaustively here, but they are well described in basic texts and in more detailed monographs, as well as in a voluminous research literature, and thus are well known to those of skill in the art. Known modifications which may be present in polypeptides of the present invention include, but are not limited to, acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cystine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination. Such modifications are well known to those of skill and have been described in great detail in the scientific literature. Several particularly common modifications including glycosylation, lipid attachment, sulfation, gamma-carboxylation of glutamic acid residues, hydroxylation and ADP-ribosylation are described in most basic texts such as PROTEINS - STRUCTURE AND MOLECULAR PROPERTIES, 2nd Ed., T. E. Creighton, W. H. Freeman and Company, New York, 1993. Detailed reviews are also available on this subject. See e.g., Wold, F., Posttranslational Protein Modifications: Perspectives and Prospects, pgs. 1-12 in POSTTRANSLATIONAL COVALENT MODIFICATION OF PROTEINS, B. C. Johnson, Ed., Academic Press, New York, 1983; Seifter *et al.,* "Analysis for protein modifications and nonprotein cofactors", *Meth. Enzymol.,* 1990, *182*:626-646 and Rattan *et al.,* "Protein Synthesis: Posttranslational Modifications and Aging", *Ann. N. Y. Acad. Sci.,* 1992, 663: 48-62.

It will be appreciated, as is well known and as noted above, that polypeptides are not always entirely linear. For instance, polypeptides may be branched as a result of ubiquitination, and they may be circular, with or without branching, generally as a result of posttranslation events, including natural processing event and events brought about by human manipulation which do not occur naturally. Circular, branched and branched circular polypeptides may be synthesized by non-translation natural processes and by entirely synthetic methods, as well.

Modifications can occur anywhere in a polypeptide, including the peptide backbone, the amino acid side-chains and the amino or carboxyl termini. In fact, blockage of the amino or carboxyl group in a polypeptide, or both, by a covalent modification, is common in naturally occurring and synthetic polypeptides and such modifications may be present in polypeptides of the present invention, as well. For instance, the amino terminal residue of polypeptides made in *E. coli,* prior to processing, almost invariably will be N-formylmethionine.

The modifications that occur in a polypeptide often will be a function of how it is made. For polypeptides made by expressing a cloned gene in a host, for instance, the nature and extent of the modifications in large part will be determined by the host cell's posttranslational modification capacity and the modification signals present in the polypeptide amino acid sequence. For instance, as is well known, glycosylation often does not occur in bacterial hosts such as *E. coli.* Accordingly, when glycosylation is desired, a polypeptide should be expressed in a glycosylating host, generally a eukaryotic cell. Insect cells often carry out the same posttranslational glycosylations as mammalian cells and, for this reason, insect cell expression systems have been developed to express efficiently mammalian proteins having the native pattems of glycosylation, *inter alia.* Similar considerations apply to other modifications.

It will be appreciated that the same type of modification may be present in the same or varying degrees at several sites in a given polypeptide. Also, a given polypeptide may contain many types of modifications.

In general, as used herein, the term polypeptide encompasses all such modifications, particularly those that are present in polypeptides synthesized by expressing a polynucleotide in a host cell.

"Variant(s)" of polynucleotides or polypeptides, as the term is used herein, are polynucleotides or polypeptides that differ from a reference polynucleotide or polypeptide, respectively. Variants in this sense are described below and elsewhere in the present disclosure in greater detail.

Variants include polynucleotides that differ in nucleotide sequence from another, reference polynucleotide. Generally, differences are limited so that the nucleotide sequences of the reference and the variant are closely similar overall and, in many regions, identical.

As noted below, changes in the nucleotide sequence of the variant may be silent. That is, they may not alter the amino acids encoded by the polynucleotide. Where alterations are limited to silent changes of this type, a variant will encode a polypeptide with the same amino acid sequence as the reference. As also noted below, changes in the nucleotide sequence of the variant may alter the amino acid sequence of a polypeptide encoded by the reference polynucleotide. Such nucleotide changes may result in amino acid substitutions, additions, deletions, fusions and truncations in the polypeptide encoded by the reference sequence, as discussed below.

Variants also include polypeptides that differ in amino acid sequence from another, reference polypeptide. Generally, differences are limited so that the sequences of the reference and the variant are closely similar overall and, in many regions, identical.

A variant and reference polypeptide may differ in amino acid sequence by one or more substitutions, additions, deletions, fusions and truncations, which may be present in any combination.

"Fusion protein" as the term is used herein, is a protein encoded by two, often unrelated, fused genes or fragments thereof. EP-A-0 464 533 (Canadian counterpart 2045869) discloses fusion proteins comprising various portions of constant region of immunoglobin molecules together with another human protein or part thereof. In many cases, employing an immunoglobulin Fc region as a part of a fusion protein is advantageous for use in therapy and diagnosis resulting in, for example, improved phamacokinetic properties (EP-A 0232 262). On the other hand, for some uses it would be desirable to be able to delete the Fc part after the fusion protein has been expressed, detected and purified. Accordingly, it may be desirable to link the components of the fusion protein with a chemically or enzymatically cleavable linking region. This is the case when the Fc portion proves to be a hindrance to use in therapy and diagnosis, for example, when the fusion protein is to be used as an antigen for immunizations. In drug discovery, for example, human proteins, such as, shIL5-α have been fused with Fc portions for use in high-throughput screening assays to identify antagonists of hIL-5. See, D. Bennett *et al., Journal of Molecular Recognition,* 1995, 8:52-58; and K. Johanson *et al., The Journal of Biological Chemistry,* 1995, *270*(16):9459-9471.

Thus, this invention also relates to genetically engineered soluble fusion proteins comprised of hRAD54, or a portion thereof, and of various portions of the constant regions of heavy or light chains of immunoglobulins of various subclasses (IgG, IgM, IgA, IgE). Preferred as an immunoglobulin is the constant part of the heavy chain of human IgG, particularly IgGl, where fusion takes place at the hinge region. In one embodiment, the Fc part can be removed simple by incorporation of a cleavage sequence which can be cleaved with blood clotting factor Xa This invention further relates to processes for the preparation of these fusion proteins by genetic engineering, and to the use thereof for diagnosis and therapy. Yet a further aspect of the invention relates to polynucleotides encoding such fusion proteins.

Membrane bound proteins are particularly useful in the formation of fusion proteins. Such proteins are generally characterized as possessing three distinct structural regions, an extracellular domain, a transmembrane domain, and a cytoplasmic domain. This invention contemplates the use of one or more of these regions as components of a fusion protein. Examples of such fusion protein technology can be found in W094/29458 and W094/22914.

"Binding molecules" (or otherwise called "interaction molecules" or "receptor component factors") refer to molecules, including receptors, that specifically bind to or interact with polypeptides of the present invention. Such binding molecules are a part of the present invention. Binding molecules may also be non-naturally occurring, such as antibodies and antibody-derived reagents that bind specifically to polypeptides of the invention.

As known in the art, "similarity" between two polypeptides is determined by comparing the amino acid sequence and its conserved amino acid substitutes of one polypeptide to the sequence of a second polypeptide. Moreover, also known in the art is "identity", which means the degree of sequence relatedness between two polypeptide or two polynucleotide sequences as determined by the identity of the match between two strings of such sequences. Both identity and similarity can be readily calculated (COMPUTATIONAL MOLECULAR BIOLOGY, Lesk, A.M., ed., Oxford University Press, New York, 1988; BIOCOMPUTING: INFORMATICS AND GENOME PROJECTS, Smith, D.W., ed., Academic Press, New York, 1993; COMPUTER ANALYSIS OF SEQUENCE DATA, PART I, Griffin, A.M., and Griffin, H.G., eds., Humana Press, New Jersey, 1994; SEQUENCE ANALYSIS IN MOLECULAR BIOLOGY, von Heinje, G., Academic Press, 1987; and SEQUENCE ANALYSIS PRIMER, Gribskov, M. and Devereux, J., eds., M Stockton Press, New York, 1991). There exist a number of methods to measure identity and similarity between two polynucleotide or polypeptide sequences, and the terms "identity" and "similarity" are well known to skilled artisans (Carillo, H., and Lipton, D., *SIAM J. Applied Math.,* 1988, 48:1073). Methods commonly employed to determine identity or similarity between two sequences include, but are not limited to, those disclosed in Guide to Huge Computers, Martin J. Bishop, ed., Academic Press, San Diego, 1994, and Carillo, H., and Lipton, D., *SIAM J. Applied Math.,* 1988, 48:1073. Preferred methods to determine identity are designed to give the largest match between the two sequences tested. Methods to determine identity and similarity are also codified in computer programs. Preferred computer program methods to determine identity and similarity between two sequences include, but are not limited to, GCS program package (Devereux, J., *et al., Nucleic Acids Research,* 1984, 12(1):387), BLAST, BLAST, FAST (Atschul, S.F. *et al., J. Molec. Biol.,* 1990, 215:403).

### DESCRIPTION OF THE INVENTION

The present invention relates to novel hRAD54 polypeptides and polynucleotides, among other things, as described in greater detail below. In particular, the invention relates to polypeptides and polynucleotides of a novel hRAD54, which is related by amino acid sequence homology to yeast RAD54. The invention relates especially to hRAD54 having the nucleotide and amino acid sequences set out in SEQ ID NOS 1 to 10.

### Polynucleotides

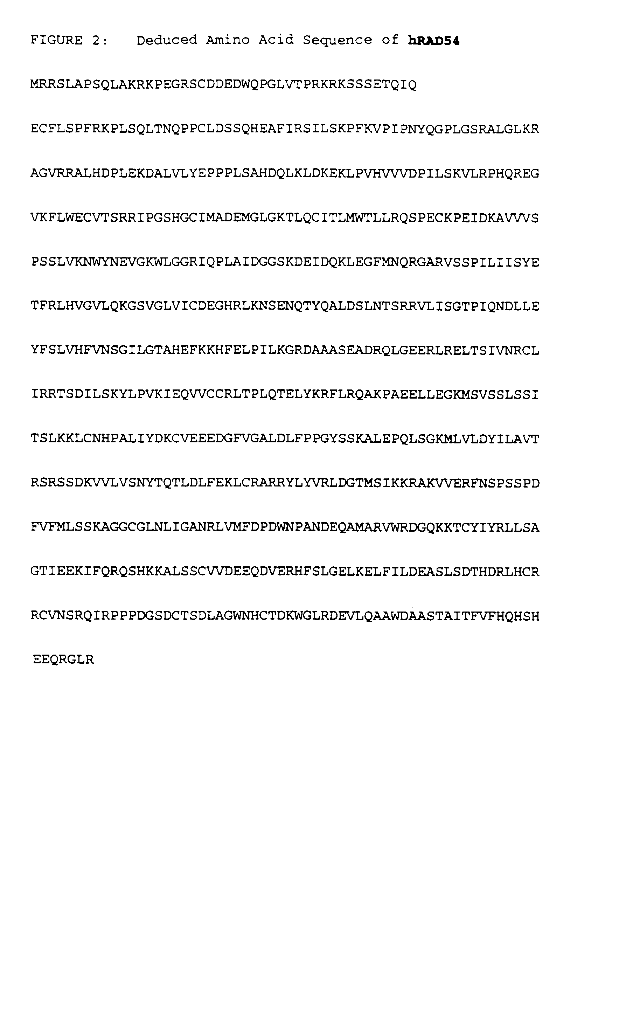

In accordance with one aspect of the present invention, there are provided isolated polynucleotides which encode the hRAD54 polypeptide having the deduced amino acid sequence of Figure 2 (SEQ ID NO: 10).

hRAD54 of the present invention belongs to the SNF2 superfamily of DNA and RNA helicases, whose members are involved in various aspects of DNA replication, repair and gene expression. The predicted protein of the hRAD54 gene contains 747 amino acids, encoding a 93kDa protein with 52% identity to the yeast RAD54 protein.

Polynucleotides of the present invention may be in the form of RNA, such as mRNA, or in the form of DNA, including, for instance, cDNA and genomic DNA obtained by cloning or produced by chemical synthetic techniques or by a combination thereof. The DNA may be double-stranded or single-stranded. Single-stranded DNA may be the coding strand, also known as the sense strand, or it may be the noncoding strand, also referred to as the anti-sense strand.

The coding sequence which encodes the polypeptide may be identical to the coding sequence of the polynucleotide shown in Figure 1, SEQ ID NOS: 1-9. It also may be a polynucleotide with a different sequence, which, as a result of the redundancy (degeneracy) of the genetic code, also encodes the polypeptide of Figure 2, SEQ ID NO: 10

Polynucleotides of the present invention which encode the polypeptide of SEQ ID: 2 may include, but are not limited to, the coding sequence for the mature polypeptide, by itself; the coding sequence for the mature polypeptide and additional coding sequences; and the coding sequence of the mature polypeptide, with or without the aforementioned additional coding sequences, together with additional, noncoding sequences. Examples of additional coding sequence include, but are not limited to, sequences encoding a leader or secretory sequence, such as a pre-, or pro- or prepro- protein sequence. Examples of additional noncoding sequences include, but are not limited to, introns and non-coding 5' and 3' sequences, such as the transcribed, non-translated sequences that play a role in transcription, and mRNA processing, including splicing and polyadenylation signals, for example, for ribosome binding and stability of mRNA. Coding sequences which provide additional functionalities may also be incorporated into the polypeptide. Thus, for instance, the polypeptide may be fused to a marker sequence, such as a peptide, which facilitates purification of the fused polypeptide. In certain preferred embodiments of this aspect of the invention, the marker sequence is a hexa-histidine peptide, such as the HA tag provided in the pQE vector (Qiagen, Inc.). As described in Gentz *et al., Proc. Natl. Acad. Sci., USA,* 1989, 86:821-824, for instance, hexa-histidine provides for convenient purification of the fusion protein. In another embodiment, the tag corresponds to an epitope derived of influenza hemagglutinin protein, which has been described by Wilson *et al.. Cell,* 1984, 37:767, for instance. Many other such tags are commercially available.

In accordance with the foregoing, the term "polynucleotide encoding a polypeptide" as used herein encompasses polynucleotides which include, by virtue of the redundancy of the genetic code, any sequence encoding a polypeptide of the present invention, particularly the hRAD54 having the amino acid sequence set out in Figure 2, SEQ ID NO: 10. The term also encompasses polynucleotides that include a single continuous region or discontinuous regions encoding the polypeptide (for example, interrupted by introns) together with additional regions, that also may contain coding and/or noncoding sequences.

The present invention further relates to variants of the herein above described polynucleotides which encode for fragments, analogs and derivatives of the polypeptide having the deduced amino acid sequence of Figure 2 (SEQ ID NO: 10). A variant of the polynucleotide may be a naturally occurring variant such as a naturally occurring allelic variant, or it may be a variant that is not known to occur naturally. Such non-naturally occurring variants of the polynucleotide may be made by mutagenesis techniques, including those applied to polynucleotides, cells or organisms.

Among variants in this regard are variants that differ from the aforementioned polynucleotides by nucleotide substitutions, deletions or additions. The substitutions, deletions or additions may involve one or more nucleotides. The variants may be altered in coding or noncoding regions or both. Alterations in the coding regions may produce conservative or non-conservative amino acid substitutions, deletions or additions.

Among the particularly preferred embodiments of the invention in this regard are polynucleotides encoding polypeptides having the amino acid sequence of hRAD54 set out in Figure 2 (SEQ ID NO: 10); variants, analogs, derivatives and fragments thereof, and fragments of the variants, analogs and derivatives.

Further particularly preferred in this regard are polynucleotides encoding hRAD54 variants, analogs, derivatives and fragments, and variants, analogs and derivatives of the fragments, which have the amino acid sequence of the hRAD54 polypeptide of Figure 2 (SEQ ID NO: 10) in which several, a few, 5 to 10, 1 to 5, 1 to 3, 2, 1 or no amino acid residues are substituted, deleted or added, in any combination. Especially preferred among these are silent substitutions, additions and deletions, which do not alter the properties and activities of the hRAD54. Also especially preferred in this regard are conservative substitutions. Most highly preferred are polynucleotides encoding polypeptides having the amino acid sequence of Figure 2 (SEQ ID NO: 10), without substitutions.

Further preferred embodiments of the invention are polynucleotides that are at least 70% identical to polynucleotide sequences set out in Figure 1 (SEQ ID NOS: 1-9), and polynucleotides which are complementary to such polynucleotides. More preferred are polynucleotides at least 80% identical to polynucleotides of SEQ ID NOS: 1-9 and polynucleotides complementary thereto. Polynucleotides at least 90% identical to the same are particularly preferred, and those with at least 95% are more particularly preferred. Furthermore, those with at least 97% are highly preferred and those with at least 98-99% are more highly preferred, with at least 99% being the most preferred.

Particularly preferred embodiments in this respect, moreover, are polynucleotides which encode polypeptides which retain substantially the same biological function or activity as the mature polypeptide encoded by polynucleotide of SEQ ID NOS: 1-9.

The present invention further relates to polynucleotides that hybridize to the herein above-described sequences. In this regard, the present invention especially relates to polynucleotides which hybridize under stringent conditions to the herein above-described polynucleotides. As herein used, the term "stringent conditions" means hybridization will occur only if there is at least 95% and preferably at least 97% identity between the sequences.

As discussed additionally herein regarding polynucleotide assays of the invention, for instance, polynucleotides of the invention as discussed above, may be used as hybridization probes for cDNA and genomic DNA, to isolate full-length cDNAs and genomic clones encoding hRAD54 and to isolate cDNA and genomic clones of other genes that have a high sequence similarity to the hRAD54 gene. Such probes generally will comprise at least 15 nucleotides. Preferably, such probes will have at least 30 nucleotides and may have at least 50 nucleotides. Particularly preferred probes will range between 30 and 50 nucleotides.

For example, the coding region of the hRAD54 gene may be isolated by screening using the known DNA sequence to synthesize an oligonucleotide probe. A labeled oligonucleotide having a sequence complementary to that of a gene of the present invention is then used to screen a library of human cDNA, genomic DNA or mRNA to determine the members of the library to which the probe hybridizes to.

The polynucleotides and polypeptides of the present invention may be employed as research reagents and materials for discovery of treatments and diagnostics to human disease, as further discussed herein relating to polynucleotide assays.

The polynucleotides may encode a polypeptide which is the mature protein plus additional amino or carboxyl-terminal amino acids, or amino acids interior to the mature polypeptide (when the mature form has more than one polypeptide chain, for instance). Such sequences may play a role in processing of a protein from precursor to a mature form, may facilitate protein trafficking, may prolong or shorten protein half-life or may facilitate manipulation of a protein for assay or production, among other things. As generally is the case *in situ,* the additional amino acids may be processed away from the mature protein by cellular enzymes.

A precursor protein, having the mature form of the polypeptide fused to one or more prosequences may be an inactive form of the polypeptide. When prosequences are removed such inactive precursors generally are activated. Some or all of the prosequences may be removed before activation. Generally, such precursors are called proproteins.

In sum, a polynucleotide of the present invention may encode a mature protein, a mature protein plus a leader sequence (which may be referred to as a preprotein), a precursor of a mature protein having one or more prosequences which are not the leader sequences of a preprotein, or a preproprotein, which is a precursor to a proprotein, having a leader sequence and one or more prosequences, which generally are removed during processing steps that produce active and mature forms of the polypeptide.

### Polypeptides

The present invention further relates to a hRAD54 polypeptide which has the deduced amino acid sequence of Figure 2, SEQ ID NO: 10.

The invention also relates to fragments, analogs and derivatives of these polypeptides. The terms "fragment," "derivative" and "analog" when referring to the polypeptide of Figure 1, mean a polypeptide which retains essentially the same biological function or activity as such polypeptide, i.e. functions as a hRAD 54, or retains the ability to bind any receptors or binding molecules even though the polypeptide does not function as the enzyme. Thus, an analog includes, for example, a proprotein which can be activated by cleavage of the proprotein portion to produce an active mature polypeptide.

The polypeptide of the present invention may be a recombinant polypeptide, a natural polypeptide or a synthetic polypeptide. In certain preferred embodiments, it is a recombinant polypeptide.

The fragment, derivative or analog of the polypeptide of Figure 2 (SEQ ID NO: 10) may be: (i) one in which one or more of the amino acid residues are substituted with a conserved or non-conserved amino acid residue (preferably a conserved amino acid residue) and such substituted amino acid residue may or may not be one encoded by the genetic code; (ii) one in which one or more of the amino acid residues includes a substituent group; (iii) one in which the mature polypeptide is fused with another compound, such as a compound to increase the half-life of the polypeptide (for example, polyethylene glycol); or (iv) one in which the additional amino acids are fused to the mature polypeptide, such as a leader or secretory sequence or a sequence which is employed for purification of the mature polypeptide or a proprotein sequence. Such fragments, derivatives and analogs are deemed to be within the scope of those skilled in the art from the teachings herein.

Among the particularly preferred embodiments of the invention in this regard are polypeptides having the amino acid sequence of hRAD54 set out in Figure 2 as SEQ ID NO: 10, variants, analogs, derivatives and fragments thereof, and variants, analogs and derivatives of the fragments. Further particularly preferred embodiments of the invention in this regard are polypeptides having the amino acid sequence of hRAD54, variants, analogs, derivatives and fragments thereof, and variants, analogs and derivatives of the fragments which retain the activity/function of this enzyme.

Among preferred variants are those that vary from a reference by conservative amino acid substitutions. Such substitutions are those that substitute a given amino acid in a polypeptide by another amino acid of like characteristics. Typically seen as conservative substitutions are the replacements, one for another, among the aliphatic amino acids Ala, Val, Leu and Ile; interchange of the hydroxyl residues Ser and Thr, exchange of the acidic residues Asp and Glu, substitution between the amide residues Asn and Gln, exchange of the basic residues Lys and Arg and replacements among the aromatic residues Phe and Tyr.

Further particularly preferred in this regard are variants, analogs, derivatives and fragments, and variants, analogs and derivatives of the fragments, having the amino acid sequence of the hRAD54 polypeptide of Figure 2 (SEQ ID NO: 10), in which several, a few, 5 to 10, 1 to 5, 1 to 3, 2, 1 or no amino acid residues are substituted, deleted or added, in any combination. Especially preferred among these are silent substitutions, additions and deletions, which do not alter the properties and activities of the enzyme. Also especially preferred in this regard are conservative substitutions. Most highly preferred are polypeptides having the amino acid sequence of Figure 2, SEQ ID NO: 10, without substitutions.

The polypeptides and polynucleotides of the present invention are preferably provided in an isolated form, and preferably are purified to homogeneity.

The polypeptides of the present invention include the polypeptide of SEQ ID NO: 10 (in particular the mature polypeptide) as well as polypeptides which have at least 70% identity to the polypeptide of SEQ ID NO: 10 and more preferably at least 90% similarity (more preferably at least 90% identity) to the polypeptide of SEQ ID NO: 10 and still more preferably at least 95% similarity (still more preferably at least 95% identity) to the polypeptide of SEQ ID NO: 10.

Fragments or portions of the polypeptides of the present invention may be employed for producing the corresponding full-length polypeptide by peptide synthesis; therefore, the fragments may be employed as intermediates for producing the full-length polypeptides. Fragments or portions of the polynucleotides of the present invention may be used to synthesize full-length polynucleotides of the present invention. Fragments may be "free-standing," i.e., not part of or fused to other amino acids or polypeptides, or they may be comprised within a larger polypeptide of which they form a part or region. When comprised within a larger polypeptide, the presently discussed fragments most preferably form a single continuous region. However, several fragments may be comprised within a single larger polypeptide. For instance, certain preferred embodiments relate to a fragment of a hRAD54 polypeptide of the present comprised within a precursor polypeptide designed for expression in a host and having heterologous pre- and pro-polypeptide regions fused to the amino terminus of the hRAD54 fragment and an additional region fused to the carboxyl terminus of the fragment. Therefore, fragments in one aspect of the meaning intended herein, refers to the portion or portions of a fusion polypeptide or fusion protein derived from hRAD54.

### Vectors, host cells, expression

The present invention also relates to vectors which contain polynucleotides of the present invention, host cells which are genetically engineered with vectors of the invention and the production of polypeptides of the invention by recombinant techniques.

Host cells can be genetically engineered to incorporate polynucleotides and express polypeptides of the present invention. For instance, polynucleotides may be introduced into host cells using well known techniques of infection, transduction, transfection, transvection and transformation. The polynucleotides may be introduced alone or with other polynucleotides. Such other polynucleotides may be introduced independently, co-introduced or introduced joined to the polynucleotides of the invention.

Thus, for instance, polynucleotides of the invention may be transfected into host cells with another, separate polynucleotide encoding a selectable marker, using standard techniques for co-transfection and selection in, for instance, mammalian cells. In this case the polynucleotides generally will be stably incorporated into the host cell genome.

Altematively, the polynucleotides may be joined to a vector containing a selectable marker for propagation in a host. The vector construct may be introduced into host cells by the aforementioned techniques. Generally, a plasmid vector is introduced as DNA in a precipitate, such as a calcium phosphate precipitate, or in a complex with a charged lipid. Electroporation may also be used to introduce polynucleotides into a host. If the vector is a virus, it may be packaged *in vitro* or introduced into a packaging cell and the packaged virus may be transduced into cells. A wide variety of techniques suitable for making polynucleotides and for introducing polynucleotides into cells in accordance with this aspect of the invention are well known and routine to those of skill in the art. Such techniques are reviewed at length in Sambrook *et al.* which is merely illustrative of the many laboratory manuals that detail these techniques.

In accordance with this aspect of the invention the vector may be, for example, a plasmid vector, a single- or double-stranded phage vector, or a single- or double-stranded RNA or DNA viral vector. Such vectors may be introduced into cells as polynucleotides, preferably DNA, by well known techniques for introducing DNA and RNA into cells. The vectors, in the case of phage and viral vectors may also be and preferably are introduced into cells as packaged or encapsidated virus by well known techniques for infection and transduction. Viral vectors may be replication competent or replication defective. In the latter case, viral propagation generally will occur only in complementing host cells.

Preferred among vectors, in certain respects, are those for expression of polynucleotides and polypeptides of the present invention. Generally, such vectors comprise cis-acting control regions effective for expression in a host operatively linked to the polynucleotide to be expressed. Appropriate trans-acting factors are either supplied by the host, supplied by a complementing vector or supplied by the vector itself upon introduction into the host.

In certain preferred embodiments in this regard, the vectors provide for specific expression. Such specific expression may be inducible expression or expression only in certain types of cells or both inducible and cell-specific expression. Particularly preferred among inducible vectors are vectors that can be induced to express a protein by environmental factors that are easy to manipulate, such as temperature and nutrient additives. A variety of vectors suitable to this aspect of the invention, including constitutive and inducible expression vectors for use in prokaryotic and eukaryotic hosts, are well known and employed routinely by those of skill in the art.

The engineered host cells can be cultured in conventional nutrient media, which may be modified as appropriate for, *inter alia,* activating promoters, selecting transformants or amplifying genes. Culture conditions, such as temperature, pH and the like, previously used with the host cell selected for expression, generally will be suitable for expression of polypeptides of the present invention as will be apparent to those of skill in the art.

A great variety of expression vectors can be used to express a polypeptide of the invention. Such vectors include chromosomal, episomal and virus-derived vectors e.g., vectors derived from bacterial plasmids, bacteriophages, yeast episomes, yeast chromosomal elements, and viruses such as baculoviruses, papova viruses, SV40, vaccinia viruses, adenoviruses, fowl pox viruses, pseudorabies viruses and retroviruses, and vectors derived from combinations thereof, such as those derived from plasmid and bacteriophage genetic elements, cosmids and phagemids. Generally, any vector suitable to maintain, propagate or express polynucleotides to produce a polypeptide in a host may be used for expression in this regard.

The appropriate DNA sequence may be inserted into the vector by any of a variety of well-known and routine techniques. In general, a DNA sequence for expression is joined to an expression vector by cleaving the DNA sequence and the expression vector with one or more restriction endonucleases and then joining the restriction fragments together using T4 DNA ligase. Procedures for restriction and ligation that can be used to this end are well known and routine to those of skill. Suitable procedures in this regard, and for constructing expression vectors using alternative techniques, which also are well known and routine to those skilled in the art, are set forth in great detail in Sambrook *et al.*

The DNA sequence in the expression vector is operatively linked to appropriate expression control sequence(s), including, for instance, a promoter to direct mRNA transcription. Representatives of such promoters include the phage lambda PL promoter, the E. *coli* lac, trp and tac promoters, the SV40 early and late promoters and promoters of retroviral LTRs, to name just a few of the well-known promoters. It will be understood that numerous other promoters useful in this aspect of the invention are well known and may be routinely employed by those of skill in the manner illustrated by the discussion and the examples herein.

In general, expression constructs will contain sites for transcription initiation and termination, and, in the transcribed region, a ribosome binding site for translation. The coding portion of the mature transcripts expressed by the constructs will include a translation initiating AUG at the beginning and a termination codon appropriately positioned at the end of the polypeptide to be translated.

In addition, the constructs may contain control regions that regulate, as well as engender, expression. Generally, in accordance with many commonly practiced procedures, such regions will operate by controlling transcription. Examples include repressor binding sites and enhancers, among others.

Vectors for propagation and expression generally will include selectable markers. Selectable marker genes provide a phenotypic trait for selection of transformed host cells. Preferred markers include, but are not limited to, dihydrofolate reductase or neomycin resistance for eukaryotic cell culture, and tetracycline or ampicillin resistance genes for culturing E. *coli* and other bacteria Such markers may also be suitable for amplification. Altematively, the vectors may contain additional markers for this purpose.

The vector containing a selected polynucleotide sequence as described elsewhere herein, as well as an appropriate promoter, and other appropriate control sequences, may be introduced into an appropriate host using a variety of well known techniques suitable for expression therein of a desired polypeptide. Representative examples of appropriate hosts include bacterial cells, such as *E. coli, Streptomvces* and *Salmonella typhimurium* cells; fungal cells, such as yeast cells; insect cells such as *Drosophila* S2 and *Spodoptera* Sf9 cells; animal cells such as CHO, COS and Bowes melanoma cells; and plant cells. Hosts of a great variety of expression constructs are well known, and those of skill will be enabled by the present disclosure to routinely select a host for expressing a polypeptide in accordance with this aspect of the present invention.

More particularly, the present invention also includes recombinant constructs, such as expression constructs, comprising one or more of the sequences described above. The constructs comprise a vector, such as a plasmid or viral vector, into which such a sequence of the invention has been inserted. The sequence may be inserted in a forward or reverse orientation. In certain preferred embodiments in this regard, the construct further comprises regulatory sequences, including, for example, a promoter, operably linked to the sequence. Large numbers of suitable vectors and promoters are known to those of skill in the art, and there are many commercially available vectors suitable for use in the present invention.

The following vectors, which are commercially available, are provided by way of example. Among vectors preferred for use in bacteria are pQE70, pQE60 and pQE-9, available from Qiagen; pBS vectors, Phagescript vectors, Bluescript vectors, pNH8A, pNH16a, pNH18A, pNH46A, available from Stratagene; and ptrc99a, pKK223-3, pKK233-3, pDR540, pRIT5 available from Pharmacia. Among preferred eukaryotic vectors are pWLNEO, pSV2CAT, pOG44, pXT1 and pSG available from Stratagene; and pSVK3, pBPV, pMSG and pSVL available from Pharmacia These vectors are listed solely by way of illustration of the many commercially available and well known vectors that are available to those of skill in the art for use in accordance with this aspect of the present invention. It will be appreciated that any other plasmid or vector suitable for, for example, introduction, maintenance, propagation or expression of a polynucleotide or polypeptide of the invention in a host may be used in this aspect of the invention.

Promoter regions can be selected from any desired gene using vectors that contain a reporter transcription unit lacking a promoter region, such as a chloramphenicol acetyl transferase ("CAT") transcription unit, downstream of a restriction site or sites for introducing a candidate promoter fragment; i.e., a fragment that may contain a promoter. As is well known, introduction into the vector of a promoter-containing fragment at the restriction site upstream of the CAT gene engenders production of CAT activity, which can be detected by standard CAT assays. Vectors suitable to this end are well known and readily available. Two examples of such vectors include pKK232-8 and pCM7. Thus, promoters for expression of polynucleotides of the present invention include not only well known and readily available promoters, but also promoters that may be readily obtained by the foregoing technique, using a reporter gene.

Among known bacterial promoters suitable for expression of polynucleotides and polypeptides in accordance with the present invention are the *E. coli* lacI and lacZ promoters, the T3 and T7 promoters, the gpt promoter, the lambda PR, PL promoters and the trp promoter.

Among known eukaryotic promoters suitable in this regard are the CMV immediate early promoter, the HSV thymidine kinase promoter, the early and late SV40 promoters, the promoters of retroviral LTRs, such as those of the Rous Sarcoma Virus("RSV"), and metallothionein promoters, such as the mouse metallothionein-I promoter.

Selection of appropriate vectors and promoters for expression in a host cell is a well known procedure and the requisite techniques for construction of expression vectors, introduction of the vector into the host and expression in the host are routine skills in the art.

The present invention also relates to host cells containing the above-described constructs. The host cell can be a higher eukaryotic cell, such as a mammalian cell, a lower eukaryotic cell, such as a yeast cell, or a prokaryotic cell, such as a bacterial cell.

Introduction of the construct into the host cell can be effected by calcium phosphate transfection, DEAE-dextran mediated transfection, cationic lipid-mediated transfection, electroporation, transduction, infection or other methods. Such methods are described in many standard laboratory manuals.

Constructs in host cells can be used in a conventional manner to produce the gene product encoded by the recombinant sequence. Altematively, the polypeptides of the invention can be synthetically produced by conventional peptide synthesizers.

Mature proteins can be expressed in mammalian cells, yeast, bacteria, or other cells under the control of appropriate promoters. Cell-free translation systems can also be employed to produce such proteins using RNAs derived from the DNA constructs of the present invention. Appropriate cloning and expression vectors for use with prokaryotic and eukaryotic hosts are described by Sambrook *et al .*

Generally, recombinant expression vectors will include origins of replication, a promoter derived from a highly-expressed gene to direct transcription of a downstream structural sequence, and a selectable marker to permit isolation of vector containing cells following exposure to the vector. Among suitable promoters are those derived from the genes that encode glycolytic enzymes such as 3-phosphoglycerate kinase ("PGK"), a-factor, acid phosphatase, and heat shock proteins, among others. Selectable markers include the ampicillin resistance gene of *E. coli* and the trp I gene of S. *cerevisiae.*

Transcnption of DNA encoding the polypeptides of the present invention by higher eukaryotes may be increased by inserting an enhancer sequence into the vector. Enhancers are cis-acting elements of DNA, usually from about 10 to 300 bp, that act to increase transcriptional activity of a promoter in a given host cell-type. Examples of enhancers include the SV40 enhancer, which is located on the late side of the replication origin at bp 100 to 270, the cytomegalovirus early promoter enhancer, the polyoma enhancer on the late side of the replication origin, and adenovirus enhancers.

A polynucleotide of the invention encoding the heterologous structural sequence of a polypeptide of the invention generally will be inserted into the vector using standard techniques so that it is operably linked to the promoter for expression. The polynucleotide will be positioned so that the transcription start site is located appropriately 5' to a ribosome binding site. The ribosome binding site will be 5' to the AUG that initiates translation of the polypeptide to be expressed. Generally, there will be no other open reading frames that begin with an initiation codon, usually AUG, and lie between the ribosome binding site and the initiation codon. Also, generally, there will be a translation stop codon at the end of the polypeptide and a polyadenylation signal and transcription termination signal appropriately disposed at the 3' end of the transcribed region.

Appropriate secretion signals may be incorporated into the expressed polypeptide for secretion of the translated protein into the lumen of the endoplasmic reticulum, the periplasmic space or the extracellular environment,. The signals may be endogenous to the polypeptide or heterologous.

The polypeptide may be expressed in a modified form, such as a fusion protein, and may include not only secretion signals but also additional heterologous functional regions. Thus, for example, a region of additional amino acids, particularly charged amino acids, may be added to the N-terminus of the polypeptide to improve stability and persistence in the host cell during purification or subsequent handling and storage. A region may also be added to the polypeptide to facilitate purification. Such regions may be removed prior to final preparation of the polypeptide. The addition of peptide moieties to polypeptides to engender secretion or excretion, to improve stability and to facilitate purification, among others, are familiar and routine techniques in the art.

Suitable prokaryotic hosts for propagation, maintenance or expression of polynucleotides and polypeptides in accordance with the invention include *Escherichia coli, Bacillus subtilis* and *Salmonella typhimurium.* Various species of *Pseudomonas, Streptomyces,* and *Staphylococcus* are also suitable hosts in this regard. Moreover, many other hosts also known to those of skill may be employed in this regard.

As a representative but non-limiting example, useful expression vectors for bacterial use can comprise a selectable marker and bacterial origin of replication derived from commercially available plasmids comprising genetic elements of the well known cloning vector pBR322 (ATCC 37017). Such commercial vectors include, for example, pKK223-3 (Pharmacia Fine Chemicals, Uppsala, Sweden) and GEMI (Promega Biotec, Madison, WI, USA). In these vectors, the pBR322 "backbone" sections are combined with an appropriate promoter and the structural sequence to be expressed.

Following transformation of a suitable host strain, the host strain is grown to an appropriate cell density. Where the selected promoter is inducible, it is induced by appropriate means (e.g., temperature shift or exposure to chemical inducer) and cells are cultured for an additional period. Cells typically then are harvested by centrifugation, disrupted by physical or chemical means, and the resulting crude extract retained for further purification.

Microbial cells employed in expression of proteins can be disrupted by any convenient method, including freeze-thaw cycling, sonication, mechanical disruption, or use of cell lysing agents. Such methods are well known to those skilled in the art.

Various mammalian cell culture systems can be employed for expression, as well. Examples of mammalian expression systems include the C127, 3T3, CHO, HeLa, human kidney 293 and BHK cell lines. and the COS-7 line of monkey kidney fibroblasts, described by *Gluzman et al., Cell,* 1981, *23*:175.

Mammalian expression vectors will comprise an origin of replication, a suitable promoter and enhancer, and any necessary ribosome binding sites, polyadenylation sites, splice donor and acceptor sites, transcriptional termination sequences, and 5' flanking non-transcribed sequences that are necessary for expression. In certain preferred embodiments, DNA sequences derived from the SV40 splice sites and the SV40 polyadenylation sites are used for required non-transcribed genetic elements.

The hRAD54 polypeptide can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, high performance liquid chromatography ("HPLC") is employed for purification. Well known techniques for refolding proteins may be employed to regenerate active conformation when the polypeptide is denatured during isolation and or purification.

Polypeptides of the present invention include naturally purified polypeptides, polypeptides produced by chemical synthetic procedures, and polypeptides produced by recombinant techniques from a prokaryotic or eukaryotic host, including, for example, bacterial, yeast, higher plant, insect and mammalian cells. Depending upon the host employed in a recombinant production procedure, the polypeptides of the present invention may be glycosylated or non-glycosylated. In addition, polypeptides of the invention may include an initial modified methionine residue, in some cases as a result of host-mediated processes.

hRAD54 polynucleotides and polypeptides may be used in accordance with the present invention for a variety of applications, particularly those that make use of the chemical and biological properties of the enzyme. Additional applications relate to diagnosis and to treatment of disorders of cells, tissues and organisms. These aspects of the invention are illustrated further by the following discussion.

### Polynucleotide assays

This invention is also related to the use of hRAD54 polynucleotides to detect complementary polynucleotides for use, for example, as a diagnostic reagent. Detection of a mutated form of hRAD54 associated with a dysfunction will provide a diagnostic tool that can add to or define diagnosis of a disease or susceptibility to a disease which results from under-expression, over-expression or altered expression of hRAD54. Individuals carrying mutations in the hRAD54 gene may be detected at the DNA level by a variety of techniques. Nucleic acids for diagnosis may be obtained from a patient's cells, such as from blood, urine, saliva, tissue biopsy or autopsy material. The genomic DNA may be used directly for detection or may be amplified enzymatically by using PCR prior to analysis. PCR (Saiki *et al., Nature,* 1986, 324:163-166). RNA or cDNA may also be used in similar fashion. As an example, PCR primers complementary to the nucleic acid encoding hRAD54 can be used to identify and analyze hRAD54 expression and mutations. For example, deletions and insertions can be detected by a change in size of the amplified product in comparison to the normal genotype. Point mutations can be identified by hybridizing amplified DNA to radiolabeled hRAD54 RNA or, radiolabeled hRAD54 antisense DNA sequences. Perfectly matched sequences can be distinguished from mismatched duplexes by RNase A digestion or by differences in melting temperatures.

Sequence differences between a reference gene and genes having mutations may also be revealed by direct DNA sequencing. In addition, cloned DNA segments may be employed as probes to detect specific DNA segments. The sensitivity of such methods can be greatly enhanced by appropriate use of PCR or other amplification methods. For example. a sequencing primer is used with double-stranded PCR product or a single-stranded template molecule generated by a modified PCR. The sequence determination is performed by conventional procedures with radiolabeled nucleotide or by automatic sequencing procedures with fluorescent-tags.

Genetic testing based on DNA sequence differences may be achieved by detection of alterations in electrophoretic mobility of DNA fragments in gels, with or without denaturing agents. Small sequence deletions and insertions can be visualized by high resolution gel electrophoresis. DNA fragments of different sequences may be distinguished on denaturing formamide gradient gels in which the mobilities of different DNA fragments are retarded in the gel at different positions according to their specific melting or partial melting temperatures (see, e.g., Myers *et al., Science,* 1985, *230:*1242).

Sequence changes at specific locations may also be revealed by nuclease protection assays, such as RNase and SI protection or the chemical cleavage method (e.g., Cotton *et al., Proc. Natl. Acad. Sci., USA,* 1985, *85:* 4397-4401).

Thus, the detection of a specific DNA sequence may be achieved by methods such as hybridization, RNase protection, chemical cleavage, direct DNA sequencing or the use of restriction enzymes, (e.g., restriction fragment length polymorphisms ("RFLP") and Southem blotting of genomic DNA.

In accordance with a further aspect of the invention, there is provided a process for diagnosing or determining a susceptibility to cancer prone syndromes, such as Xeroderma Pigmentosum and Bloom syndrome, Werner's syndromes, and X-linked Mental Retardation with alpha-thalassemia Syndrome (ATR-X); as well as cancers, such as that of the breast, among others. A mutation in the hRAD54 gene may be indicative of a susceptibility to cancer prone syndromes, such as Xeroderma Pigmentosum and Bloom syndrome, Wemer's syndromes, and X-linked Mental Retardation with alpha-thalassemia Syndrome (ATR-X); as well as cancers, such as that of the breast, among others; and the nucleic acid sequences described above may be employed in an assay for ascertaining such susceptibility. Thus, for example, the assay may be employed to determine a mutation in a hRAD54 gene, as herein described, such as a deletion, truncation, insertion, frame shift, etc., with such mutation being indicative of a susceptibility to a hyperproliferative disease, among others.

The invention provides a process for diagnosing diseases, particularly, cancer prone syndromes, such as Xeroderma Pigmentosum and Bloom syndrome, Werner's syndromes, and X-linked Mental Retardation with alpha-thalassemia Syndrome (ATR-X); as well as cancers, such as that of the breast, among others; comprising determining from a sample derived from a patient an abnormally decreased or increased level of expression of polynucleotide having the sequence of Figure 1, SEQ ID NOS: 1-9. Decreased or increased expression of polynucleotide can be measured using any of the methods well known in the art for the quantitation of polynucleotides, such as, for example, PCR, RT-PCR, RNase protection, Northern blotting and other hybridization methods.

In addition to more conventional gel-electrophoresis and DNA sequencing, mutations can also be detected by *in situ* analysis.

It is also the intention of this invention to provide the following sets of primers (Table I) for PCR genomic DNA, such as for single-strand conformational polymorphism (SSCP) analysis to detect mutation in hRAD sequences:

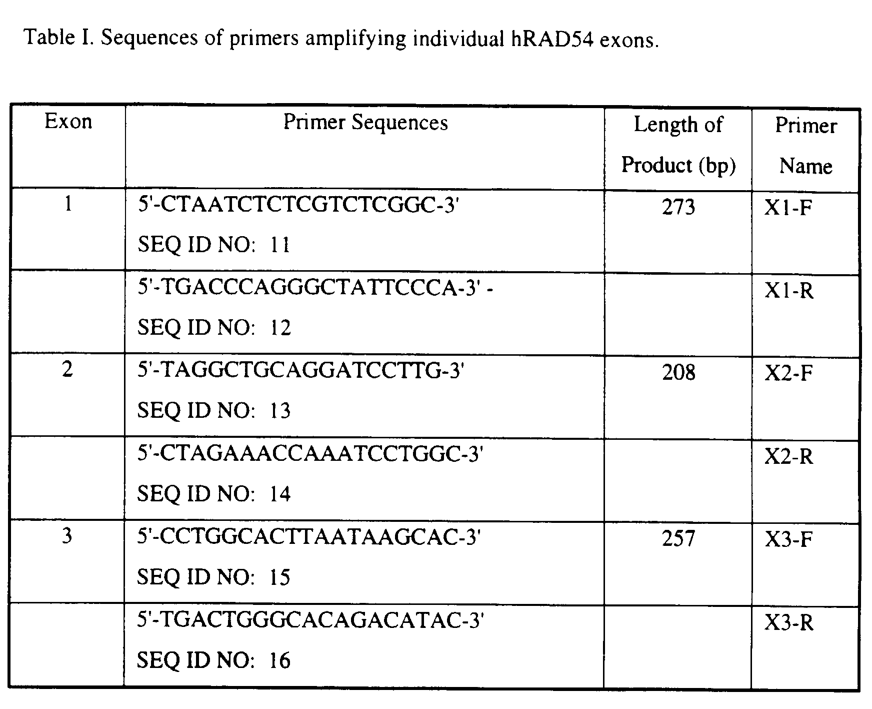

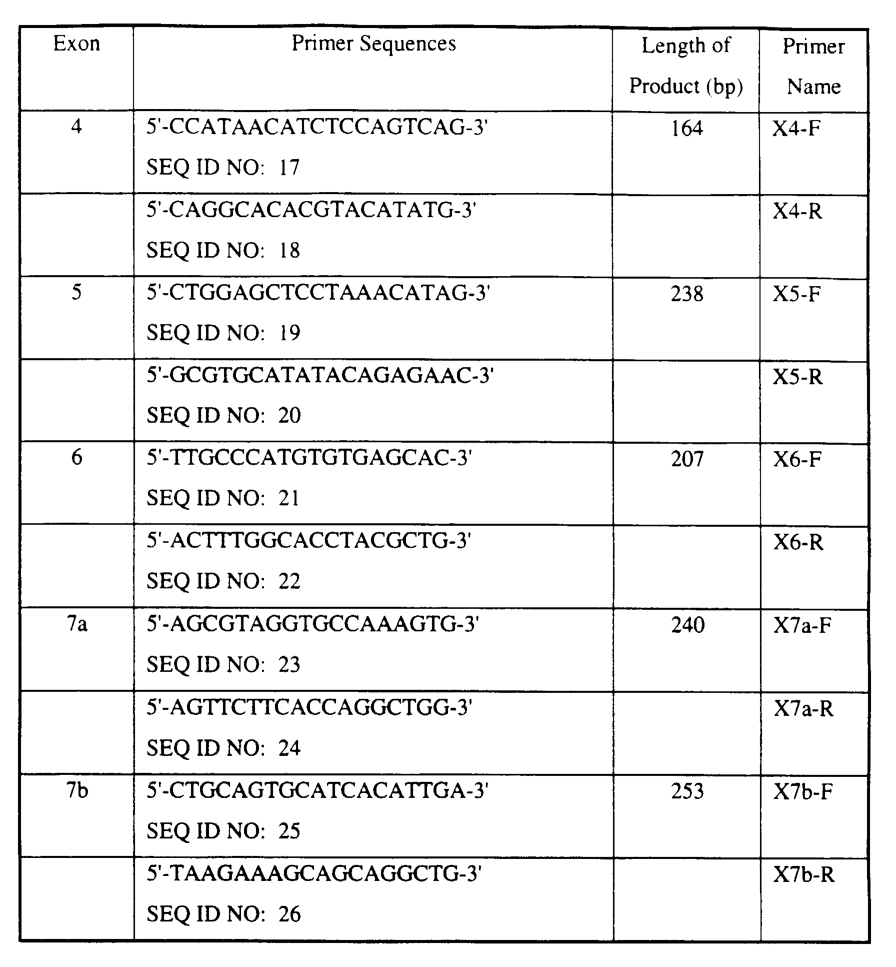

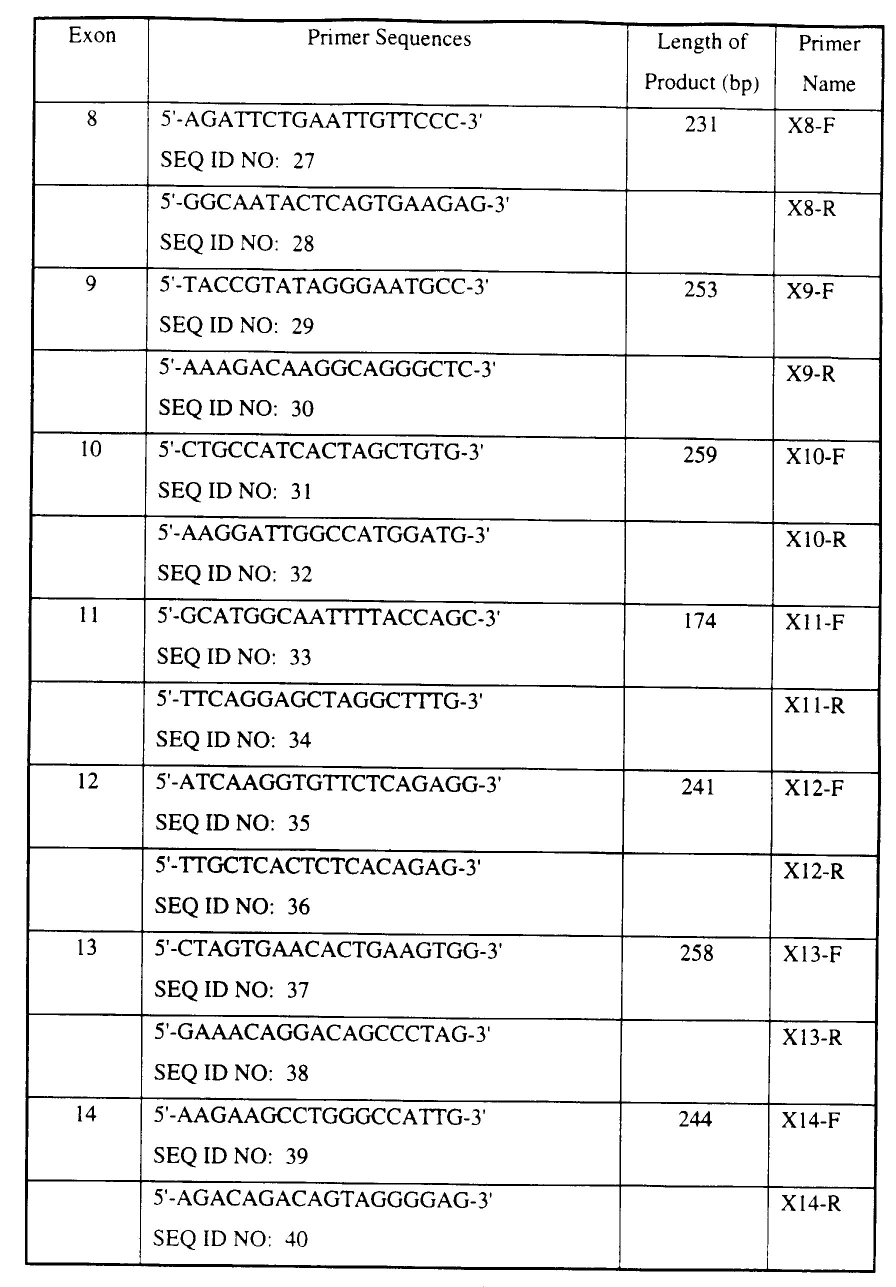

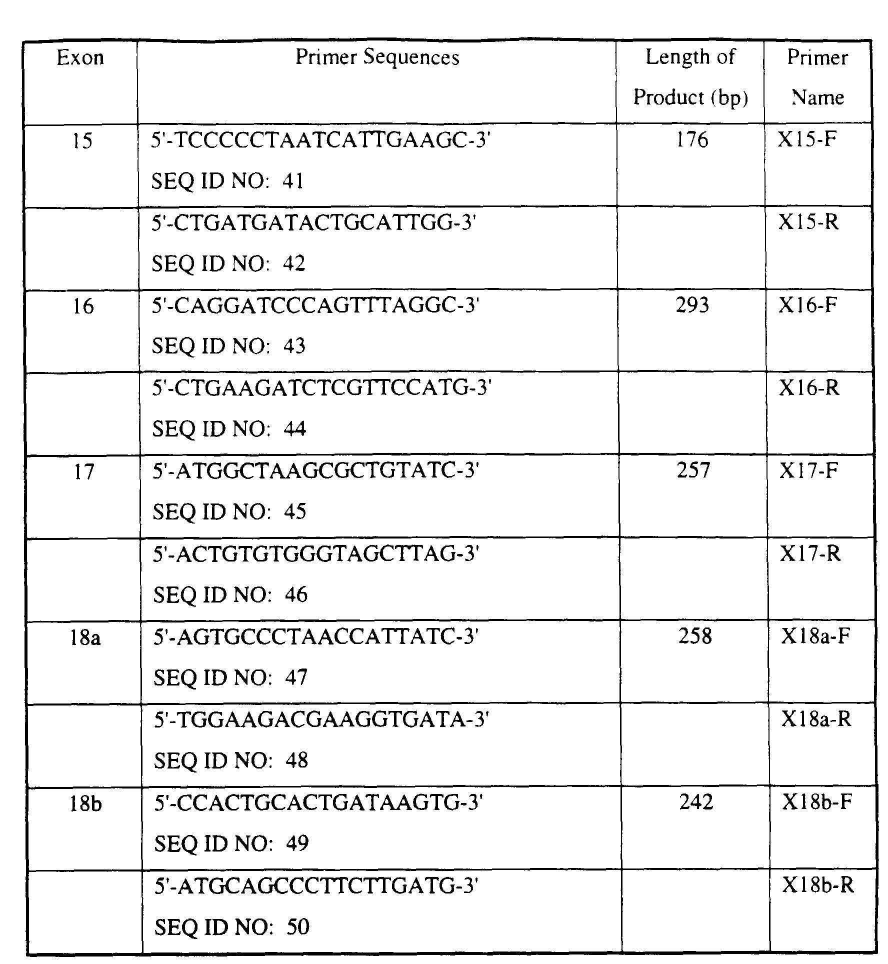

### Polypeptide assays

The present invention also relates to diagnostic assays for detecting levels of hRAD54 protein in cells and tissues. Such assays may be quantitative or qualitative, Thus, for instance, a diagnostic assay in accordance with the invention for detecting over-expression of hRAD54 protein compared to normal control tissue samples may be used to detect the presence of cancer prone syndromes, such as Xeroderma Pigmentosum and Bloom syndrome, Wemer's syndromes, and X-linked Mental Retardation with alpha-thalassemia Syndrome (ATR-X); as well as cancers, such as that of the breast, among others. Assay techniques that can be used to determine levels of a protein, such as a hRAD54 protein of the present invention, in a sample derived from a host are well-known to those of skill in the art. Such assay methods include radioimmunoassays. competitive-binding assays, Westem Blot analysis and enzyme linked immunosorbent assays (ELISAs). Among these, ELISAs are frequently preferred. An ELISA assay initially comprises preparing an antibody specific to hRAD54, preferably a monoclonal antibody. In addition a reporter antibody generally is prepared which binds to the monoclonal antibody. The reporter antibody is attached to a detectable reagent such as a radioactive, fluorescent or enzymatic reagent, in this example, horseradish peroxidase enzyme.

To carry out an ELISA, a sample is removed from a host and incubated on a solid support, e.g. a polystyrene dish, that binds the proteins in the sample. Any free protein binding sites on the dish are then covered by incubating with a non-specific protein such as bovine serum albumin. The monoclonal antibody is then incubated in the dish during which time the monoclonal antibodies attach to any hRAD54 proteins attached to the polystyrene dish. Unbound monoclonal antibody is washed out with buffer. The reporter antibody linked to horseradish peroxidase is placed in the dish resulting in binding of the reporter antibody to any monoclonal antibody bound to hRAD54 protein. Unattached reporter antibody is then washed out. Reagents for peroxidase activity, including a colorimetric substrate are then added to the dish. Immobilized peroxidase, linked to hRAD54 through the primary and secondary antibodies, produces a colored reaction product. The amount of color developed in a given time period indicates the amount of hRAD54 protein present in the sample. Quantitative results typically are obtained by reference to a standard curve.

A competition assay may also be employed wherein antibodies specific to hRAD54 attached to a solid support and labeled hRAD54 and a sample derived from the host are passed over the solid support. The amount of detected label attached to the solid support can be correlated to a quantity of hRAD54 in the sample.

### Antibodies

The polypeptides, their fragments or other derivatives, or analogs thereof, or cells expressing them can also be used as immunogens to produce antibodies thereto. These antibodies can be, for example, polyclonal or monoclonal antibodies. The present invention also includes chimeric, single chain, and humanized antibodies, as well as Fab fragments, or the product of a Fab expression library. Various procedures known in the art may be used for the production of such antibodies and fragments.

Antibodies generated against polypeptides corresponding to a sequence of the present invention can be obtained by various means well known to those of skill in the art. For example, in one embodiment, the polypeptide is directly injected into an animal, preferably a nonhuman. The antibody so obtained will then bind the polypeptide itself. In this embodiment, even a sequence encoding only a fragment of the polypeptide can be used to generate antibodies binding the whole native polypeptide. Such antibodies can then be used to isolate the polypeptide from tissues expressing that polypeptide.

For preparation of monoclonal antibodies, any technique which provides antibodies produced by continuous cell line cultures can be used. Examples include the hybridoma technique (Kohler, G. and Milstein, C., *Nature,* 1975, 256:495497), the trioma technique, the human B-cell hybridoma technique (Kozbor *et al., Immunology Today,* 1983, 4:72) and the EBV-hybridoma technique (Cole *et al.,* MONOCLONAL ANTIBODIES AND CANCER THERAPY, pg. 77-96, Alan R. Liss, Inc., 1985).

Techniques described for the production of single chain antibodies (U.S. Patent No. 4,946,778) can also be adapted to produce single chain antibodies to immunogenic polypeptide products of this invention. Also, transgenic mice, or other organisms including other mammals, may be used to express humanized antibodies to immunogenic polypeptide products of this invention.

The above-described antibodies may be employed to isolate or to identify clones expressing the polypeptide or to purify the polypeptide of the present invention by attachment of the antibody to a solid support for isolation and/or purification by affinity chromatography.

Antibodies against hRAD54 may also be employed to inhibit cancer prone syndromes, such as Xeroderma Pigmentosum and Bloom syndrome, Werner's syndromes, and X-linked Mental Retardation with alpha-thalassemia Syndrome (ATR-X); as well as cancers, such as that of the breast, among others.

### Binding molecules and assays

hRAD54 can also be used to isolate proteins which interact with it; this interaction can be a target for interference. Inhibitors of protein-protein interactions between hRAD54 and other factors could lead to the development of pharmaceutical agents for the modulation of hRAD54 activity.

Thus, this invention also provides a method for identification of binding molecules to hRAD54. Genes encoding proteins for binding molecules to hRAD54 can be identified by numerous methods known to those of skill in the art, for example, ligand panning and FACS sorting. Such methods are described in many laboratory manuals such as, for instance, Coligan *et al.,* CURRENT PROTOCOLS IN IMMUNOLOGY 1, Chapter 5, 1991.

For example, the yeast two-hybrid system provides methods for detecting the interaction between a first test protein and a second test protein, *in vivo,* using reconstitution of the activity of a transcriptional activator. The method is disclosed in U.S. Patent No. 5,283,173; reagents are available from Clontech and Stratagene. Briefly, hRAD54 cDNA is fused to a Gal4 transcription factor DNA binding domain and expressed in yeast cells. cDNA library members obtained from cells of interest are fused to a transactivation domain of Gal4. cDNA clones which express proteins which can interact with hRAD54 will lead to reconstitution of Gal4 activity and transactivation of expression of a reporter gene such as Gal4-lacZ.

An alternative method involves screening of λgt11, λZAP (Stratagene) or equivalent cDNA expression libraries with recombinant hRAD54. Recombinant hRAD54 protein or fragments thereof are fused to small peptide tags such as FLAG, HSV or GST. The peptide tags can possess convenient phosphorylation sites for a kinase such as heart muscle creatine kinase or they can be biotinylated. Recombinant hRAD54 can be phosphorylated with ³²[P] or used unlabeled and detected with streptavidin or antibodies against the tags. λgt11cDNA expression libraries are made from cells of interest and are incubated with the recombinant hRAD54, washed and cDNA clones which interact with hRAD54 isolated. Such methods are routinely used by skilled artisans. See, e.g., Sambrook *et al.*

Another method is the screening of a mammalian expression library. In this method, cDNAs are cloned into a vector between a mammalian promoter and polyadenylation site and transiently transfected in COS or 293 cells. Forty-eight hours later, the binding protein is detected by incubation of fixed and washed cells with labeled hRAD54. In a preferred embodiment, the hRAD54 is iodinated, and any bound hRAD54 is detected by autoradiography. See Sims *et al., Science,* 1988, *241:585-589* and McMahan *et al., EMBO J.,* 1991, *10:2821-2832.* In this manner, pools of cDNAs containing the cDNA encoding the binding protein of interest can be selected and the cDNA of interest can be isolated by further subdivision of each pool followed by cycles of transient transfection, binding and autoradiography. Alternatively, the cDNA of interest can be isolated by transfecting the entire cDNA library into mammalian cells and panning the cells on a dish containing hRAD54 bound to the plate. Cells which attach after washing are lysed and the plasmid DNA isolated, amplified in bacteria, and the cycle of transfection and panning repeated until a single cDNA clone is obtained. See Seed *et al, Proc. Natl. Acad. Sci. USA,* 1987, 84:3365 and Aruffo *et al., EMBO J.,* 1987, 6:3313. If the binding protein is secreted, its cDNA can be obtained by a similar pooling strategy once a binding or neutralizing assay has been established for assaying supernatants from transiently transfected cells. General methods for screening supernatants are disclosed in Wong *et al., Science,* 1985, *228*:810-815.

Another method involves isolation of proteins interacting with hRAD54 directly from cells. Fusion proteins of hRAD54 with GST or small peptide tags are made and immobilized on beads. Biosynthetically labeled or unlabeled protein extracts from the cells of interest are prepared, incubated with the beads and washed with buffer. Proteins interacting with hRAD54 are eluted specifically from the beads and analyzed by SDS-PAGE. Binding partner primary amino acid sequence data are obtained by microsequencing. Optionally, the cells can be treated with agents that induce a functional response such as tyrosine phosphorylation of cellular proteins. An example of such an agent would be a growth factor or cytokine such as interleukin-2.

Another method is immunoaffinity purification. Recombinant hRAD54 is incubated with labeled or unlabeled cell extracts and immunoprecipitated with anti-human antibodies. The immunoprecipitate is recovered with protein A-Sepharose and analyzed by SDS-PAGE. Unlabelled proteins are labeled by biotinylation and detected on SDS gels with streptavidin. Binding partner proteins are analyzed by microsequencing. Further, standard biochemical purification steps known to those skilled in the art may be used prior to microsequencing.

Yet another alternative method involves screening of peptide libraries for binding partners. Recombinant tagged or labeled hRAD54 is used to select peptides from a peptide or phosphopeptide library which interact with hRAD54. Sequencing of the peptides leads to identification of consensus peptide sequences which might be found in interacting proteins.

### Agonists and antagonists - assays and molecules

The hRAD54 of the present invention may be employed in a screening process for compounds which activate (agonists) or inhibit activation (antagonists) of this enzyme.

Potential antagonists also include proteins which are closely related to hRAD54 , i.e. a fragment of the enzyme, which have lost enzymatic activity.

A potential antagonist also includes an antisense construct prepared through the use of antisense technology. Antisense technology can be used to control gene expression through triple-helix formation or antisense DNA or RNA, both methods of which are based on binding of a polynucleotide to DNA or RNA. For example, the 5' coding portion of the polynucleotide sequence, which encodes for the mature polypeptides of the present invention, is used to design an antisense RNA oligonucleotide of from about 10 to 40 base pairs in length. A DNA oligonucleotide is designed to be complementary to a region of the gene involved in transcription (triple helix -see Lee *et al., Nucl. Acids Res.,* 1979*,* 6:3073; Cooney *et al., Science, 1988. 241:456;* and Dervan *et al., Science,* 1991, *251*:1360), thereby preventing transcription and production of the hRAD54. The antisense RNA oligonucleotide hybridizes to the mRNA *in vivo* and blocks translation of the mRNA molecule into the enzyme (antisense - see Okano, *J. Neurochem.,* (1991) 56:560; Oligodeoxynucleotides as Antisense Inhibitors of Gene Expression, CRC Press, Boca Raton, FL (1988)). The oligonucleotides described above can also be delivered to cells such that the antisense RNA or DNA is expressed *in vivo* to inhibit production of hRAD54.

Another potential antagonist is a small molecule which binds to the enzyme, making it inaccessible to ligands such that normal biological activity is prevented. Examples of small molecules include, but are not limited to, small peptides or peptide-like molecules.

Potential antagonists also include soluble forms of hRAD54 e.g., fragments of the enzyme, which bind to ligands, thus preventing the ligand from interacting with membrane bound hRAD54.

Antagonists for hRAD54 may be employed for a variety of therapeutic and prophylactic purposes for such cancer prone syndromes, such as Xeroderma Pigmentosum and Bloom syndrome, Werner's syndromes, and X-linked Mental Retardation with alpha-thalassemia Syndrome (ATR-X); as well as cancers, such as that of the breast, among others.

This invention additionally provides a method of treating an abnormal condition related to an excess of hRAD54 activity which comprises administering to a subject an inhibitor compound (antagonist) as hereinabove described along with a pharmaceutically acceptable carrier in an amount effective to inhibit activation of the enzyme, or by inhibiting a second signal, and thereby alleviating the abnormal condition.

In general, agonists for hRAD54 are employed for therapeutic and prophylactic purposes for such diseases or disorders where induction of a cell into a hyperproliferative state is preferred.

The invention also provides a method of treating abnormal conditions related to an under-expression of hRAD54 and its activity, which comprises administering to a subject a therapeutically effective amount of a compound which activates (agonist) the enzyme, to thereby alleviate the abnormal condition.

### Compositions and Kits

The soluble form of hRAD54, and compounds which activate or inhibit such enzyme, may be employed in combination with a suitable pharmaceutical carrier. Such compositions comprise a therapeutically effective amount of the polypeptide or compound. and a pharmaceutically acceptable carrier or excipient. Such carriers include, but are not limited to, saline, buffered saline, dextrose, water, glycerol, ethanol, and combinations thereof. The formulation should suit the mode of administration. Selection of an appropriate carrier in accordance with the mode of administration is routinely performed by those skilled in the art.

The invention further relates to pharmaceutical packs and kits comprising one or more containers filled with one or more of the ingredients of the aforementioned compositions of the invention.

### Administration

Polypeptides and other compounds of the present invention may be employed alone or in conjunction with other compounds, such as therapeutic compounds.

The pharmaceutical compositions may be administered in any effective, convenient manner including, for instance, administration by topical, oral, anal, vaginal, intravenous, intraperitoneal, intramuscular, subcutaneous, intranasal or intradermal routes, among others.

The pharmaceutical compositions generally are administered in an amount effective for treatment or prophylaxis of a specific indication or indications. In general, the compositions are administered in an amount of at least about 10 µg/kg body weight. In most cases they will be administered in an amount not in excess of about 8 mg/kg body weight per day. Preferably, in most cases, the administered dose is from about 10 µg/kg to about 1 mg/kg body weight, daily. It will be appreciated that optimum dosage will be determined by standard methods for each treatment modality and indication, taking into account the indication, its severity, route of administration, complicating conditions and the like.

### Gene therapy

hRAD54 polynucleotides, polypeptides, agonists and antagonists that are polypeptides may be employed in accordance with the present invention by expression of such polypeptides in treatment modalities often referred to as "gene therapy."

Thus, for example, cells from a patient may be engineered with a polynucleotide, such as a DNA or RNA, to encode a polypeptide *ex vivo.* The engineered cells can then be provided to a patient to be treated with the polypeptide. In this embodiment, cells may be engineered *ex vivo,* for example, by the use of a retroviral plasmid vector containing RNA encoding a polypeptide of the present invention. Such methods are well-known in the art and their use in the present invention will be apparent from the teachings herein.

Similarly, cells may be engineered *in vivo* for expression of a polypeptide *in vivo* by procedures known in the art. For example, a polynucleotide of the invention may be engineered for expression in a replication defective retroviral vector, as discussed above. The retroviral expression construct may then be isolated and introduced into a packaging cell transduced with a retroviral plasmid vector containing RNA encoding a polypeptide of the present invention such that the packaging cell now produces infectious viral particles containing the gene of interest. These producer cells may be administered to a patient for engineering cells *in vivo* and expression of the polypeptide *in vivo.* These and other methods for administering a polypeptide of the present invention should be apparent to those skilled in the art from the teachings of the present invention.

Retroviruses from which the retroviral plasmid vectors herein above mentioned may be derived include, but are not limited to, Moloney Murine Leukemia Virus, Spleen Necrosis Virus, Rous Sarcoma Virus, Harvey Sarcoma Virus, Avian Leukosis Virus, Gibbon Ape Leukemia Virus, Human Immunodeficiency Virus, Adenovirus, Myeloproliferative Sarcoma Virus, and Mammary Tumor Virus. In a preferred embodiment, the retroviral plasmid vector is derived from Moloney Murine Leukemia Virus.

Such vectors will include one or more promoters for expressing the polypeptide. Suitable promoters which may be employed include, but are not limited to, the retroviral LTR; the SV40 promoter; and the human cytomegalovirus (CMV) promoter described in Miller *et al., Biotechniques,* 1989, *7*:980-990. Cellular promoters such as eukaryotic cellular promoters including, but not limited to, the histone, RNA polymerase III, and β-actin promoters can also be used. Additional viral promoters which may be employed include, but are not limited to, adenovirus promoters, thymidine kinase (TK) promoters, and B19 parvovirus promoters. The selection of a suitable promoter will be apparent to those skilled in the art from the teachings contained herein.

The nucleic acid sequence encoding the polypeptide of the present invention will be placed under the control of a suitable promoter. Suitable promoters which may be employed include, but are not limited to, adenoviral promoters, such as the adenoviral major late promoter; or heterologous promoters, such as the cytomegalovirus (CMV) promoter; the respiratory syncytial virus (RSV) promoter; inducible promoters, such as the MMT promoter, the metallothionein promoter; heat shock promoters; the albumin promoter; the ApoAI promoter; human globin promoters; viral thymidine kinase promoters, such as the Herpes Simplex thymidine kinase promoter; retroviral LTRs (including the modified retroviral LTRs herein above described); the β-actin promoter; and human growth hormone promoters. The promoter may also be the native promoter which controls the gene encoding the polypeptide.

The retroviral plasmid vector is employed to transduce packaging cell lines to form producer cell lines. Examples of packaging cells which may be transfected include, but are not limited to, the PE501, PA317, Y-2, Y-AM, PA12, T19-14X, VT-19-17-H2. YCRE, YCRIP. GP+E-86, GP+envAm12, and DAN cell lines as described in Miller, A., *Human Gene Therapy.* 1990, 1:5-14. The vector may be transduced into the packaging cells through any means known in the art. Such means include, but are not limited to, electroporation, the use of liposomes, and CaPO₄ precipitation. In one altemative, the retroviral plasmid vector may be encapsulated into a liposome, or coupled to a lipid, and then administered to a host.

The producer cell line will generate infectious retroviral vector particles, which include the nucleic acid sequence(s) encoding the polypeptides. Such retroviral vector particles may then be employed to transduce eukaryotic cells, either *in vitro* or *in vivo.* The transduced eukaryotic cells will express the nucleic acid sequence(s) encoding the polypeptide. Eukaryotic cells which may be transduced include, but are not limited to, embryonic stem cells, embryonic carcinoma cells, as well as hematopoietic stem cells, hepatocytes, fibroblasts, myoblasts, keratinocytes, endothelial cells, and bronchial epithelial cells.

### EXAMPLES

The present invention is further described by the following examples. The examples are provided solely to illustrate the invention by reference to specific embodiments. These exemplifications, while illustrating certain specific aspects of the invention, do not portray the limitations or circumscribe the scope of the disclosed invention.

Certain terms used herein are explained in the foregoing glossary.

All examples are carried out using standard techniques, which are well known and routine to those of skill in the art, except where otherwise described in detail. Routine molecular biology techniques of the following examples can be carried out as described in standard laboratory manuals, such as Sambrook *et al.*

### Database search for novel DNA repair enzymes .

The amino acid sequence from the yeast DNA excision repair enzyme RAD54 (genebank number M63232) was used to screen the Human Genome Sciences (HGS) computer database with the TFASTA computer software designed by the Genetics computer Group (GCG, University of Winsconsin). The HGS database contains nucleotide sequence information of expressed sequence tags (ESTs) (Adams, M. D., Kelley, J. M., Gocayne, J. D., Dubnick, M., Polymeropoulos, M. H., Xiao, H., Merril, C. R., Wu, A., Olde, B., Moreno, R. F., and et al. *Complementary DNA sequencing: expressed sequence tags and human genome project,* Science. *252:* 1651-6, 1991) which identify a diverse collection of cDNAs derived from more than 400 cDNA libraries. One EST (designated C2) was found to have significant homology but not identity to the RAD54 protein sequence: 60% identity in a 126 amino acid overlap. At the DNA sequence level, the EST was found to have a 51% identity to the yeast RAD54 over 492 bases. In a separate comparison, the amino acid sequence from the human DNA excision repair enzyme ERCC6 (genebank number L04791) (Troelstra, C., van Gool, A., de Wit, J., Vermeulen, W., Bootsma, D., and Hoeijmakers, J. H. ERCC6, a member of a subfamily of putative helicases, is involved in Cockayne's syndrome and preferential repair of active genes, Cell. 71: 939-53, 1992) was also used to screen the HGS database with TFASTA. The same EST homologous to yeast RAD54 was found to have significant homology but not identity to the ERCC6 protein sequence (36.9% identity in a 84 amino acid overlap). At the DNA sequence level, the EST was found to have a 64% identity to the human ERCC6 over 220 bases. The EST sequence was compared to the HGS database using the FASTA program (GCG, University of Wisconsin), and two additional ESTs were found to give overlapping sequence identity. The three ESTs were assembled into a single consensus sequence of 401 bases with the SEQMAN routine of the LaserGene software package (DNAstar).

### cDNA cloning -

Primers from the 5' and 3' end of the C2 EST were used on peripheral blood cDNA to generate a PCR-derived probe for the screening of a normal testis IDR2 cDNA library (Clontech) by conventional plaque hybridization. Eight clones were initially isolated and excised in pDR2 plasmid according to the manufacturer's instructions. Sequence analysis of the 8 positive clones revealed that one of them contained a 3.2 Kb insert encoding the entire open reading frame. Double strand sequencing of this clone was performed by a cycle sequencing program using the dye-deoxynucleotide kit and Taq DNA polymerase (Perkin Elmer). Nucleotide sequences were determined by an automated Applied Biosystem Sequencer model 377.

### Northern Analysis -

A probe derived from the insert of the cDNA clone containing the entire hRAD54 open reading frame was used to hybridize a multi-tissue Northem blot (Clontech) according to the manufacturer's protocol. Filter was washed with 0.5X SSC-0.1X SDS for 1.5 h at 60° and subjected to autoradiography for 16 h.

### Chromosomal Mapping -

Primers C2f (AGCCCTGACTTTGTCTTCA) (SEQ ID NO: 51) and C2r (GCTTGTTCATCCATTGGCT) (SEQ ID NO: 52), derived from C2 EST, were able to amplify a 120 bp product on human genomic DNA. They were used for PCR screening of the GeneBridge 4 radiation hybrid mapping panel (Research Genetics). PCR reactions were carried out in a 10 ml final volume with the following conditions: 95°C, 5 min., then 35 cycles of 94°C, 15 sec; 57°C, 20 sec; 72°C, 30 sec; followed by 5 min. of extension at 72°C. PCR products were electrophoresed on a 1.5% agarose gel and visualized by ethidium bromide staining. The result of the screening was analyzed with the statistical program RHMAP, and linkage to marker D1S443, on chromosome lp32, LOD score>3.0, was given.

### Isolation of Genomic Clones -

Primers C2f and C2r were used for PCR screening of a human genomic BAC library (Research Genetics). Four positives clones were identified, and DNA from two of them was purified with Qiagen Plasmid Midi kit, and used as template for sequencing analysis.

### Determination of exon-intron boundaries and intron sizes -

Primers derived from the cDNA were used for bidirectional sequencing of the genomic BAC clones. Exon-intron boundaries were identified by the presence of consensus splice junctions at sites were the sequence of the genomic product differed from the hRAD54 cDNA sequence. The computer program FASTA was used for the comparison of the sequences. Introns size was determined by direct sequencing or by gel electrophoresis of inter-exon PCR products.

### SSCP Analysis -

Genomic DNAs from 17 breast cancer cell lines and 20 sporadic breast tumors were analyzed for mutations. Primers amplifying individual exons were designed on the basis of intronic sequences. PCR reactions were performed in 10 ml of final volume with 20 ng of genomic DNA using 0.5 units of Taq DNA polymerase, 200 mM each of dATP dGTP, dTTP, 2.0 mM of dCTP and (a-32P) dCTP at 0.5 mCi/reaction. Cycles were as follow: 95°C, 5 min., then 22 cycles of 94°C, 15 sec; 57°C, 20 sec; 72°C, 20 sec; followed by 5 min. of extension at 72°C. SSCP products were run on a 0.5X MDE gel (FMC BioProducts) for 16 hours, and exposed for visualization by autoradiography on KODAK X-AR5 film.

## Claims

1. A DNA sequence for use in analyzing a sample for mutation of DNA encoding hRAD54, comprising:
a DNA sequence of at least 15 and no more than 30 consecutive bases of the DNA sequences consisting of SEQ ID NO: 1, 2, 3, 4, 5, 6, 7, 8, and 9.

2. A combination of DNA fragments comprising SEQ ID NOS. 1-9.

3. An isolated polynucleotide comprising a member selected from the group consisting of:
(a) a polynucleotide having at least a 70% identity to a polynucleotide of SEQ ID NO 1;
(b) a polynucleotide having at least a 70% identity to a polynucleotide of SEQ ID NO 2;
(c) a polynucleotide having at least a 70% identity to a polynucleotide of SEQ ID NO 3;
(d) a polynucleotide having at least a 70% identity to a polynucleotide of SEQ ID NO 4;
(e) a polynucleotide having at least a 70% identity to a polynucleotide of SEQ ID NO 5;
(f) a polynucleotide having at least a 70% identity to a polynucleotide of SEQ ID NO 6;
(g) a polynucleotide having at least a 70% identity to a polynucleotide of SEQ ID NO 7;
(h) a polynucleotide having at least a 70% identity to a polynucleotide of SEQ ID NO 8;
(i) a polynucleotide having at least a 70% identity to a polynucleotide of SEQ ID NO 9;
(j) a polynucleotide which is complementary to the polynucleotide of (a), (b), (c), (d), (e), (f), (g), (h), or (i); and
(k) a polynucleotide comprising at least 15 contiguous bases of the polynucleotide of (a), (b), (c), (d), (e), (f), (g), (h), or (i).

4. An isolated polynucleotide comprising a member selected from the group consisting of:
(a) polynucleotide of SEQ ID NO 1;
(b) polynucleotide of SEQ ID NO 2;
(c) polynucleotide of SEQ ID NO 3;
(d) polynucleotide of SEQ ID NO 4;
(e) polynucleotide of SEQ ID NO 5;
(f) polynucleotide of SEQ ID NO 6;
(g) polynucleotide of SEQ ID NO 7;
(h) polynucleotide of SEQ ID NO 8;
(i) polynucleotide of SEQ ID NO 9;
(j) a polynucleotide which is complementary to the polynucleotide of (a), (b), (c), (d), (e), (f), (g), (h), or (i); and
(k) a polynucleotide comprising at least 15 contiguous bases of the polynucleotide of (a), (b), (c), (d), (e), (f), (g), (h), or (i).

5. A vector comprising a DNA of Claim 3.

6. A host cell comprising the vector of Claim 5.

7. A process for producing a polypeptide comprising expressing from the host cell of Claim 6 a polypeptide encoded by said DNA.

8. A process for producing a cell which expresses a polypeptide comprising transforming or transfecting the cell with the vector of Claim 5 such that the cell expresses the polypeptide encoded by the human gDNA contained in the vector.

9. An agonist to the polypeptide of SEQ ID NO: 10.

10. An antibody against the polypeptide of SEQ ID NO: 10.

11. An antagonist to the polypeptide of SEQ ID NO: 10.

12. A method for the treatment of a patient having need of hRAD54 comprising administering to the patient a therapeutically effective amount of the polypeptide of SEQ ID NO: 10.

13. The method of Claim 12 wherein said therapeutically effective amount of the polypeptide is administered by providing to the patient DNA encoding said polypeptide and expressing said polypeptide *in vivo.*

14. A method for the treatment of a patient having need to inhibit hRAD54 polypeptide comprising administering to the patient a therapeutically effective amount of the antagonist of Claim 11.

15. A method for the treatment of a patient having need to augment hRAD54 polypeptide comprising administering to the patient a therapeutically effective amount of the agonist of Claim 9.

16. A process for diagnosing a disease or a susceptibility to a disease related to expression of the polypeptide of SEQ ID NO: 10 comprising determining a mutation in the nucleic acid sequence encoding said polypeptide.

17. A diagnostic process comprising analyzing for the presence of the polypeptide of claim SEQ ID NO: 10 in a sample derived from a host.

18. A method for determining the genetic predisposition to cancer in an individual comprising:
detecting hRAD54 mutations in a sample from the individual by PCR with AN oligonucleotide primer having nucleotide sequence selected from the group consisting of SEQ ID NOS: 11-50.

19. An oligonucleotide having a sequence selected from the group consisting of SEQ ID NOS: 11-50.


SEQUENCE LISTING

(1) GENERAL INFORMATION

(i) APPLICANTS: SmithKline Beecham Corporation
Thomas Jefferson University

(ii) TITLE OF THE INVENTION: HRAD54

(iii) NUMBER OF SEQUENCES: 52

(iv) CORRESPONDENCE ADDRESS:
(A) ADDRESSEE: SmithKline Beecham, Corporate Intellectual Property
(B) STREET: Two New Horizons Court
(C) CITY: Brentford
(D) STATE: Middlesex
(E) COUNTRY: United Kingdom
(F) POST CODE: TW8 9EP

(v) COMPUTER READABLE FORM:
(A) MEDIUM TYPE: Diskette
(B) COMPUTER: IBM Compatible
(C) OPERATING SYSTEM: DOS
(D) SOFTWARE: FastSEQ Version 1.5

(vi) CURRENT APPLICATION DATA:
(A) APPLICATION NUMBER:
(B) FILING DATE:
(C) CLASSIFICATION:

(vii) PRIOR APPLICATION DATA:
(A) APPLICATION NUMBER: 60/030,676
(B) FILING DATE: November 13, 1996

(viii) ATTORNEY/AGENT INFORMATION:
(A) NAME: CONNELL, Anthony Christopher
(B) GENERAL AUTHORISATION NUMBER: 5630 (1st applicant only)
(C) REFERENCE/DOCKET NUMBER: ATG50032

(ix) TELECOMMUNICATION INFORMATION:
(A) TELEPHONE: +44 127 964 4395
(B) TELEFAX: +44 181 975 6294
(C) TELEX:

(2) INFORMATION FOR SEQ ID NO:1:

(i) SEQUENCE CHARACTERISTICS:
(A) LENGTH: 1212 base pairs
(B) TYPE: nucleic acid
(C) STRANDEDNESS: single
(D) TOPOLOGY: linear

(ii) MOLECULE TYPE: cDNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:1:

GGTCTTGGCG GGTCGGTGAG TCTTGGCGGC TGTTAACGCG CGCTTTGGGA ACAGGAAGGT 60
TGAGAGAGAG GTGCTGGGGT CTGCGTCTAT CTCTGTCGCT CTTTTCAGCC CCTCCTGGTA 120
TTCCCCTCCT AACCTGGGTT TTTTACACGC CCGCGTGGCT TCCTGCTCGA CCTCCCTGAG 180
TCTGATCCTG GTTTCCACCT CCAGCCCTGG GAAATTTCCT TTCTCCAGAC TCGCCCTCCC 240
CACCCGGGCC TCGGACTTTC ACCCCAGCTT CTCTCTCCTG GCCAGTGATT ACCCACCCCC 300
AATCCCACCC CGCCCCGCCG CGCAACTACC TCCTCCCTTC ACCCGGACTG GGACCATCAT 360
CCCCACTCCA CTCCGCCCAG TCTGGGACTC CACCTGCCTC CTCCCCAATC CCACACTAAT 420
CTCTGCTTGG TCTCTTCCTC TTTGGCCTAA TCTCTCGTCT CGGCTTATTG GGGACGGCCA 480
CTCTCACAGT TTGGTTCCAA ACACCAGTTC CTGGATGGAT TCCCGCCATC CATGCCCCCT 540
CTTTAATTAG CCGGTCCTCT CAATAATGTA GCAGCCCCCT CTACAGATTA GACCCTGGTC 600
CTACACTCTT AGCCGCTGCC TGCTTTTGAC CTTTGGCTCA TGGGTACTTG ACGTTTTAAA 660
CTCCTAGGCC CAGGATGGTA AGTGTGGGCC TAGGGGAGAC TGGGAATAGC CCTGGGTCAG 720
GGTCTAGTAG GCCTAGGCTG CAGGATCCTT GCAGGCACTG TTTCTGTTCT CCCTTTACAG 780
AGGAGGAGCT TGGCTCCCAG CCAGCTGGCC AAGAGAAAAC CTGAAGGCAG GTCCTGTGAT 840
GATGAAGACT GGCAACCTGG CCTAGTGGTG AGCACTCAAG GGGACAGGGA AGGTGGGTAG 900
AGCTGTTTGG ACAGAAAAGA AGCCAGGATT TGGTTTCTAG GGTTACATAN CCAATTCCAG 960
ATGCCTCTTT TGTATGTTCA ATAAACTTTT AGTGAGTACT TACTATGTGC CAGGCATTGA 1020
ACAAGATTAG TAAAATCTTT ATCCTCAGGG TTAGTCACTA NAAGCTAATA TAGCCTCAGT 1080
TTTCCTGGTG GGAAAAGATA AGCAAAAGAG CCAGTTGGAT TGGACCTTTT GACTCTGCTT 1140
GGAAAAAAGG GCTACACAAC AGCAATTATC AGCTGGCTCT TTTGCTCTTT GTCCCCTGCC 1200
CAGTGTCACA GA 1212

(2) INFORMATION FOR SEQ ID NO:2:

(i) SEQUENCE CHARACTERISTICS:
(A) LENGTH: 435 base pairs
(B) TYPE: nucleic acid
(C) STRANDEDNESS: single
(D) TOPOLOGY: linear

(ii) MOLECULE TYPE: cDNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:2:

AATTTAGCAC AGTGCCTGGC ACTTAATAAG CACTTCAAGT GTTAGTAACC ATTATGGTGA 60
TTTCTGTGGT TTTCTAGACT TCACCTTTCC CAATTCTCTC TCCTAGACTC CTAGGAAACG 120
GAAATCCAGC AGTGAGACCC AGATCCAGGA GTGTTTCCTG TCTCCTTTTC GGAAACCTTT 180
GAGTCAGCTA ACCAATCAAC CACCTTGTCT GGACAGCAGT CAGCATGTAA GCCAGAACTG 240
CAACCTGCAT GTGTATGTCT GTGCCCAGTC ACTCAGCCTA GGTAGACTCC TGTCCCTGTA 300
CACTCCCTCA GTGCTTTCTA GAGTGGCCAG AAGACACACA TCTGTAGGGG CTTTGGACCT 360
GCCCACAGCT GCTGGGGCCT GCTTAGAGAG GTGTGCCTGA CTTGTGCCTG GTTTATAGTA 420
GCTACATGGG CACCA 435

(2) INFORMATION FOR SEQ ID NO:3:

(i) SEQUENCE CHARACTERISTICS:
(A) LENGTH: 359 base pairs
(B) TYPE: nucleic acid
(C) STRANDEDNESS: single
(D) TOPOLOGY: linear

(ii) MOLECULE TYPE: cDNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:3:

TCCCCTGGAT ATTTGTAGAA CCTTACATAA AGCCTCGAAG ATGGATGCTG TTGTACAAAA 60
CCTAATGTGA AGCATATCAT GATATTGTCC CATAACATCT CCAGTCAGTC CTGAATCCTT 120
GTTTCTCCTT TCTTTTTAGG AAGCATTTAT TCGAAGCATT TTGTCAAAGC CTTTCAAAGT 180
CCCCATTCCA AATTATCAAG GTAAAATGGA GGTTTTTCTG TTTTTGAAAT CAGTCATATG 240
TACGTGTGCC TGAATAAATT AAAAACCTGC TTTTGTAAAT ACAAGCTTAG CTGGGAATTG 300

AAGGTGCCAG TCCAGCGGTG GCTGTGTTTG GGGTGACCCT CCTGAGGCCT GCATGAAAA 359

(2) INFORMATION FOR SEQ ID NO:4:

(i) SEQUENCE CHARACTERISTICS:
(A) LENGTH: 2008 base pairs
(B) TYPE: nucleic acid
(C) STRANDEDNESS: single
(D) TOPOLOGY: linear

(ii) MOLECULE TYPE: cDNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:4:

CTGAGGCAGG AGAATTGCTT GAACCTGGGA GGCAGAGGTT GCAGTGAGCC GAGATTGTGC 60
CACTGCACTC CAGCCTGGGC AACAAAGTGA GACTCCATCT CAAAAAAAAA AAAAAGCTGA 120
ATGGGATAAT GGTCATATAG AGATGCCCAA ACTGAGTTTG GAGGCATTCT CAGAGAGAGA 180
GGGTCATATG GGAGCTCGGG AAAGGTTATG TGAAGGACAA GTATTTGATC ATGGGCTTGC 240
TGGAGCTCCT AAACATAGAT TCAGGTGACG GAATGATTAT TGGTTTGTAG GTCCTCTGGG 300
CTCTCGAGCA TTGGGCCTGA AAAGGGCTGG GGTCCGCCGG GCCCTCCATG ACCCCCTGGA 360
AAAAGATGCC TTGGTTCTGT ATGAGCCTCC CCCGCTGAGC GCTCATGACC AGCTGAAGCT 420
TGACAAGTAT GTGCACTGGT ATTTCATAAG CAGTTTTGGT TCTCTGTATA TGCACGCATA 480
CTTGGGAGGG CAGAGGGTAT TTTGGTGTGA TTTCTTTTTA GGATTGACAT ATGTTTTCAG 540
AGAGTAGATG ATTGGGGAAG GAGACTGCCT GGGGAAGAGC CTGTGGTCAT GTCCAGTGAG 600
TGGGCATTCC TCATCCCCTC CTGCCTTCCC AGGATGCTCA CTCAGGAGGA GGAGTGATTG 660
AGGTGATGAC ACAGGGAACC CATCCCTCTC TGAGCTAACT TTCTAACTCC TAAAGTAGGA 720
ACTTGCTATG GTTTTACGAT TTATCACCCA AGAAGGTCTT CTTTTAGGCA GCTGCCTCTG 780
TTGCCTCCGG ATCACCAGGA CACAGCTTCC TGAGGGTGCT CCCTTGCCCA TGTCTGAGCA 840
CGCTGTTTTC TTTGCTGTGT TTTCTCAGGG AGAAACTCCC TGTCCATGTG GTTGTTGACC 900
CTATTCTCAG TAAGGTTTTG CGGCCTCATC AGAGAGAGGT AAATGAGGGT GAGGGGAACG 960
AGGTATGGGC TATGGGCTGA GCCTGGGAGA CTACCATCCC TGGGACAGCA GCAGCGTAGG 1020
TGCCAAAGTG GACTGAAGGC TGTTATTCTC TAGGGAGTGA AATTCCTGTG GGAGTGTGTC 1080
ACCAGTCGGC GCATCCCTGG CAGCCATGGC TGCATCATGG CTGATGAGAT GGGCCTAGGA 1140
AAGACGCTGC AGTGCATCAC ATTGATGTGG ACACTTTTAC GCCAGAGTCC AGAGTGCAAG 1200
CCAGAAATTG ACAAGGCAGT GGTGGTGTCG CCTTCCAGCC TGGTGAAGAA CTGGTACAAT 1260
GAGGTTGGGA AATGGCTCGG AGGGAGGATC CAACCTCTGG CCATCGATGG AGGATCTAAG 1320
GATGAAATAG ACCAAAAGCT GGGTACGGAG CCCTATTCNA AGATGGCTGC ACTCTCCTGC 1380
ACAGCCTGCT GCTTTCTTAC GTGTATGCTC ATTTATGGCC AGGGTGGAGG GCAATGCAGG 1440
GGTAGAAGAA AAGAGAATTT CCATTGAAAA TAGTTGAAGT GGAGTCAGTT GTTTCCAGGC 1500
TAAATTAAAG AACTGTCTAA TTGTTTTTTT TGTTTTTTTT TTTCTCAAAA GATTCTGAAT 1560
TGTTCCCTTT ACACCTTTTC TGTTGTAGAA GGATTCATGA ACCAGCGTGG AGCCAGGGTG 1620

TCTTCTCCCA TCCTCATCAT TTCCTATGAG ACCTTCCGCC TTCATGTTGG AGTCCTCCAG 1680
AAAGGAAGTG TTGGTCTGGT CATATGTGAC GAGGTACTTG ACTCTCACCA GTCTGGGTGG 1740
TANGAGAAAA TCTGTCAAAA TCTCTTCACT GAGTATTGCC TTCCAAGGGC TGTGCTACTC 1800
ACTGGGGAAT GCTGAAGACA GGATGCTGCC TTGGAGACAA GTGACAGGGA NAGCCTGTGC 1860
ATACCAAAAT ACAAGATACT GTCTGCCAAA AGAGGGCTGA AACAATGCTT ANAACCCTGA 1920
GGAGAGTGAG ATACTGGGGG CTGATTGGCT TTGTTGCCTC CGGATAGTAT TCCTACACTA 1980
AAGAAGGACC TTGAAGGGTG GGTGGCAA 2008

(2) INFORMATION FOR SEQ ID NO:5:

(i) SEQUENCE CHARACTERISTICS:
(A) LENGTH: 840 base pairs
(B) TYPE: nucleic acid
(C) STRANDEDNESS: single
(D) TOPOLOGY: linear

(ii) MOLECULE TYPE: cDNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:5:

AGCCTCGACC TCCTGGACTC AAGCGATCTT CCCATCTCAG CCTCCCAAGT AGCTGGGGCT 60
ATGGGTGTAA GCTACTATGC CCAGCTAATT TTTGTATGTT TTGTAGAGAT GGGGTTTCAC 120
CACACTGGCC AGGCTGGTCT GGAACTCCTG GGCTCAAGTG ATCCACCTGT CTCGGCCTCC 180
CCAAAGTGCT GGGATTACAG GCATGAGCCA CGGCGCCCGG CCTGGAATGT TTTTGACTCC 240
TCTTTTCCTG TCTACATGAG ACTTTGCTAC CGTATAGGGA ATGCCACATT GCGCTCTGAA 300
TAAGGATTCT CTTGGCAGGG ACACAGGCTC AAGAACTCTG AGAATCAGAC TTACCAAGCC 360
CTGGACAGCT TGAACACCAG CCGGCGGGTG CTCATCTCCG GAACTCCCAT CCAGAATGAT 420
CTGCTTGAGT ATTTCAGCTT GGTACATTTT GTTAATTCCG GCATCCTAGG TAAGAACTAG 480
CCTTGTTTGC CACATCAGAG AGGAGCCCTG CCTTGTCTTT GGGAGTATAA CTCCAGCTGA 540
GGGAGAGAAA GAAGAGAATG CTAGTTATAT GTGGGTCACT AGCATTCTTG TAGGAAGGCT 600
TCTCTTGTCA GGGGAGTAGC AAAGCCATTG GGGAGGCCTT GGATCTGCTG AGCAACTCAG 660
CAAAGCCCAT GTTGGCCATT TTTCCATTGC AGAGGTGGCA GGGGCTATAC AGGGCAATGG 720
TGTCATGATG AGCTGAGGGT GGAATTATTA TTTTTGTTCC TCAGATCCTA GCTCCCTTAG 780
AAGAGTTTTG GCCTCAGAGC AAGCTCTGTC TTGGGCAGAC GTGTGTGTTT TAAAAATCAT 840

(2) INFORMATION FOR SEQ ID NO:6:

(i) SEQUENCE CHARACTERISTICS:
(A) LENGTH: 509 base pairs
(B) TYPE: nucleic acid
(C) STRANDEDNESS: single

(D) TOPOLOGY: linear

(ii) MOLECULE TYPE: cDNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:6:

AGGTAGTCTG CCATCACTAG CTGTGGGAAA ATCATTCCTT ATCCACATTC TTCTGTTTTC 60
CTTCTCTGCC TGNGTGTAGG GACTGCCCAT GAATTCAAGA AGCATTTTGA ATTGCCAATT 120
TTGAAGGGTC GAGACGCTGC TGCTAGTGAG GCAGACAGGC AGCTAGGAGA GGAGCGGCTG 180
CGGGAGCTCA CCAGCATTGT GAATAGGTAA TGACCTTAAG CGAAGTCATT AGAATTGCCT 240
CCCAAACCAT CCATGGCCAA TCCTTTGGGG GCTTTGCCTC TCTAGAGCCC TCAAAGGCTG 300
GGATTCTAGG AAGGGAGTGG GTTCTGTGTC CTAACTATGG CCACTGAATA AACAAGGGAG 360
TCTTGGAGGT GAAGTGCCTG CTTTCTTTCT GGCTTGGGCT CTGTGGTCTG GTTTTGGTCT 420
GTTTATCAGC CTGCTGAGGA AGCTTTTCTC TGATGCTGCT GGACTTCTTG AGCAAAGTAT 480
ACCTTGGAGC ATTACTAGTA TTTCAGAAG 509

(2) INFORMATION FOR SEQ ID NO:7:

(i) SEQUENCE CHARACTERISTICS:
(A) LENGTH: 572 base pairs
(B) TYPE: nucleic acid
(C) STRANDEDNESS: single
(D) TOPOLOGY: linear

(ii) MOLECULE TYPE: cDNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:7:

CTTGGTCCTT TGGGTGGTAG CTTTTATTCC AGTAGTTAGC ATGGCAATTT TACCAGCCTC 60
TTGCCTTTTT ATCCTGTTTT CTCTAGATGC CTGATACGGA GGACTTCTGA TATCCTTTCT 120
AAATATCTGC CTGTGAAGAT TGAGCAGGTC GTTTGTTGTA GGTACTGAAC TCAACTGAAA 180
GATGTGGAGT GGGTCAAAGC CTAGCTCCTG AAGCATGGAT GGGATCTCAA GGTGTTCTCA 240
GAGGGCAGGA GGGTGGTCTA GTTTTTCCAC TGACCCAGCT GCCTTTTTTA GGCTGACACC 300
CCTTCAGACT GAGTTATACA AGAGGTTTCT GAGACAAGCC AAACCGGCAG AAGAATTGCT 360
TGAGGGCAAG ATGAGTGTGT CTTCCCTTTC TTCCATCACC TCGCTAAAGA AGCTTTGTAA 420
TCGTGAGTTG GGCTTGTGTC CTGGTGTCCT CTGTGAGAGT GAGCAAGGGA GGGTAGGTTC 480
CTGTAGTGGC CTGGCAACCC TCACTAAAAC TCGTCCAGAG TCATCCTCAT AGAGGCCACA 540

TGTGATTCCA ACCCTACCCA AGGCATGAAC CC 572

(2) INFORMATION FOR SEQ ID NO:8:

(i) SEQUENCE CHARACTERISTICS:
(A) LENGTH: 1851 base pairs
(B) TYPE: nucleic acid
(C) STRANDEDNESS: single
(D) TOPOLOGY: linear

(ii) MOLECULE TYPE: cDNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:8:

CTGTTATAGC TCCCTTCTTG TTGGGAGAAC ACATTAGTGA GTGAGATTGT CCATCCATCG 60
TCCCAGATGG CACCGACTAT ATCCTGTTGG AATTTGGTAG GATGAAGATC AAGCAAGTCT 120
GATGTTGAGG AAGGCCTTTT GGGCTATGCT TCTAGAGGGA GTTCTAGTGA ACACTGAAGT 180
GGAAACTTCA GAAAGCAAAG TATCTGGGTT TTGTTTTGTT TTCTCCCAGA TCCAGCTCTA 240
ATCTATGATA AGTGTGTGGA AGAGGAGGAT GGCTTTGTGG GTGCCTTGGA CCTCTTCCCT 300
CCTGGTTACA GCTCTAAGGC CCTGGAGCCC CAGCTGTCAG GTGACCCTTT TCATACCAGT 360
ATTTGGGCTT CTCTAGGAGG AGGGTGGGAG ATTTTTTTTT GTGCTAGGGC TGTCCTGTTT 420
CAAGGCAGGA TATCAAGAGT AAGAAGCCTG GGCCATTGGG ACAGCCAGTA GGGGACTGCT 480
GGTTGCTGCT TCTTCCCAGG TAAGATGCTG GTCCTGGATT ATATTCTGGC GGTGACCCGA 540
AGCCGTAGCA GTGACAAAGT AGTGCTGGTG TCGAATTACA CCCAGACTTT GGATCTCTTT 600
GAGAAGCTGT GCCGTGCCCG AAGGTAGGGA AGATCCTAAC CAGGATGCCA AAGGGGGATA 660
TACCCCTCCC CTACTGTCTG TCTATTTCTG ATTTGTGGAT GTGGGCCAAG ATCTGGGCAC 720
ATAAGGGCTT TCCCTGGAGA TATCTTCCCT TATTGCTATG ATGGCCTCAG CTCTATCTCC 780
AAGTAGAAGA TCCCCTAGTC CTTCAGGTAC TCCCTTAGGA AAGAGTCTTG GCCTCTAGCT 840
CCCTGCCCCT GCCTTTGGGA GCTTAGGGCC TCAAGCATGG TTATTTCAGT CCCTTCAGTG 900
GCTCTCCAAG GGACTCTGCT TTCTTGGGTC TTTTTAAAAA AATTTTTATT TTTAATTTTT 960
TTTCCCCCTA ATCATTGAAG CTTTATTTTC TTGGGTCTCG AATCCCCCTT CAGGTACTTA 1020
TACGTCCGCC TGGATGGCAC GATGTCCATT AAGAAGCGAG CCAAGGTTGT AGAACGCTTC 1080
AATAGTCCAT CGGTAAATGC ACATCCCCGT CCCCACACCA CCAATGCAGT ATCATCAGAA 1140
TTAAGGAATG ATAGAGAAGC TATAAGGGGT TACCTTGATT TTTTTTTTTT TTAATTCCCC 1200
TAGTGGATAA AGTAGGATTT GTCTGGTTGG TGATGGCTGG GCTGGAAACT GACTGTGTGT 1260
GTTGGGATTG GCTGGCTGGT GAGCAGATAA ACTGGTGGTT TTCACAATTG GTACTGAGTA 1320
GTATAGAGGC ATGAGGGAGG AGCTGGTTGG GCTGAGCAGG ATCCCAGTTT AGGCTATAAG 1380
GGTTCCTTTT CTCCTGTTTC TTCTCTTTTC CAGAGCCCTG ACTTTGTCTT CATGCTGAGC 1440
AGCAAAGCTG GGGGCTGTGG CCTCAATCTC ATTGGGGCTA ACCGGCTGGT CATGTTTGAC 1500
CCTGACTGGA ACCCAGCCAA TGATGAACAA GCCATGGCCC GGGTCTGGCG AGATGGTCAA 1560
AAGAAGACTT GCTATATCTA CCGCCTGCTG TCTGTAAGGA TGGTGATAGT AGTCATAGTA 1620

GGGGTGGGTC ATGGAACGAG ATCTTCAGGA CCATCTTGTT CCTTAGTGCC TCTCTTTGGC 1680
CTTTCTGCCT CTAGCAGTAG CCAAGCTATG GGCCCAGAAG TGAGTCTTTT ATTCTTCTGT 1740
TCTCCCTCCC CTTCTCTCCA CCCCATTCCC TCAAACCATT CTTACTGTGC CATCTGGACA 1800
CCTGTTCAGT ACATCCTTTT CACAGGACAC CTGCTTCCCT CATACATGAT T 1851

(2) INFORMATION FOR SEQ ID NO:9:

(i) SEQUENCE CHARACTERISTICS:
(A) LENGTH: 996 base pairs
(B) TYPE: nucleic acid
(C) STRANDEDNESS: single
(D) TOPOLOGY: linear

(ii) MOLECULE TYPE: cDNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:9:

CCTGACGGGA TTTAGCCAGG CCCTGGGACC CTTCATTAGT ATAGAAAGTA GCCTGGGTGG 60
GCAGACTATT CATGTCATGT TCTCAGGAGA CAGACTGGGA AAATGGACCT CAGCTGAGTA 120
GAGATAATGT TCTGGGACTC ATACTTTTTA TTCATCTCCT CTTCTCGGGT AGCTGTAGTT 180
TCAACCCTTT GGTTTTCCTG CTCCCTTTTT ATGAGGATGG CTAAGCGCTG TATCTTTTGA 240
CATTCCCCAC CTCCTCTTTC CCCAGGCAGG GACCATTGAG GAGAAGATCT TCCAGCGTCA 300
GAGCCACAAG AAGGCACTGA GCAGCTGTGT GGTGGATGAG GAGCAGGATG TAGAGCGCCA 360
CTTCTCTCTG GGCGAGTTGA AGGAGCTGTT TATCCTGGAT GAAGCTAGCC TCAGTGACAC 420
ACATGACAGG TGGGGAAGTG CCCTAACCAT TATCTCTAAG CTACCCACAC AGTATGGAGA 480
TGGGATCTGT ANTGACTTCA GCTGTGCCTT CTGTCCCTAG GTTGCACTGC CGACGTTGTG 540
TCAACAGCCG TCAGATCCGG CCACCCCCTG ATGGTTCTGA CTGCACTTCA GACCTGGCAG 600
GGTGGAACCA CTGCACTGAT AAGTGGGGGC TCCGGGATGA GGTACTCCAG GCTGCCTGGG 660
ATGCTGCCTC CACTGCTATC ACCTTCGTCT TCCACCAGCG TTCTCATGAG GAGCAGCGGG 720
GCCTCCGCTG ATAACCAGCT GGTCTGGGTG TAGCTCTTAG AGGAAGGAGA TAGGGAAAAG 780
GGGCTCCTTG CTCCACAGGG CCCTGTTGAA TTTTGTTCTT TGGGAGAAAA TCATCAAGAA 840
GGGCTGCATG ATGTTTGCCC AAAATTTATT TTATAAGAAA AACTTTTTTG GTTAAAAAAA 900
AGAATAAAGG TATGAAAGGG TTTGAGGCCT GCAGCAGCAG TGTGGTAAGC CCATCANGGC 960
AATAAGCCCC CTACTACTTC TCTAAAGCCT GGGTGC 996

(2) INFORMATION FOR SEQ ID NO:10:

(i) SEQUENCE CHARACTERISTICS:
(A) LENGTH: 750 amino acids
(B) TYPE: amino acid
(C) STRANDEDNESS: single

(D) TOPOLOGY: linear

(ii) MOLECULE TYPE: peptide
(iii) HYPOTHETICAL: NO
(iv) ANTISENSE: NO
(v) FRAGMENT TYPE: N-terminal
(vi) ORIGINAL SOURCE:

(xi) SEQUENCE DESCRIPTION: SEQ ID NO:10:

Met Arg Arg Ser Leu Ala Pro Ser Gln Leu Ala Lys Arg Lys Pro Glu
1               5                   10                  15
Gly Arg Ser Cys Asp Asp Glu Asp Trp Gln Pro Gly Leu Val Thr Pro
            20                  25                  30
Arg Lys Arg Lys Ser Ser Ser Glu Thr Gln Ile Gln Glu Cys Phe Leu
        35                  40                  45
Ser Pro Phe Arg Lys Pro Leu Ser Gln Leu Thr Asn Gln Pro Pro Cys
    50                  55                  60
Leu Asp Ser Ser Gln His Glu Ala Phe Ile Arg Ser Ile Leu Ser Lys
65                  70                  75                  80
Pro Phe Lys Val Pro Ile Pro Asn Tyr Gln Gly Pro Leu Gly Ser Arg
                85                  90                  95
Ala Leu Gly Leu Lys Arg Ala Gly Val Arg Arg Ala Leu His Asp Pro
            100                 105                 110
Leu Glu Lys Asp Ala Leu Val Leu Tyr Glu Pro Pro Pro Leu Ser Ala
        115                 120                 125
His Asp Gln Leu Lys Leu Asp Lys Glu Lys Leu Pro Val His Val Val
    130                 135                 140
Val Asp Pro Ile Leu Ser Lys Val Leu Arg Pro His Gln Arg Glu Gly
145                 150                 155                 160
Val Lys Phe Leu Trp Glu Cys Val Thr Ser Arg Arg Ile Pro Gly Ser
                165                 170                 175
His Gly Cys Ile Met Ala Asp Glu Met Gly Leu Gly Lys Thr Leu Gln
            180                 185                 190
Cys Ile Thr Leu Met Trp Thr Leu Leu Arg Gln Ser Pro Glu Cys Lys
        195                 200                 205
Pro Glu Ile Asp Lys Ala Val Val Val Ser Pro Ser Ser Leu Val Lys
    210                 215                 220
Asn Trp Tyr Asn Glu Val Gly Lys Trp Leu Gly Gly Arg Ile Gln Pro
225                 230                 235                 240
Leu Ala Ile Asp Gly Gly Ser Lys Asp Glu Ile Asp Gln Lys Leu Glu
                245                 250                 255
Gly Phe Met Asn Gln Arg Gly Ala Arg Val Ser Ser Pro Ile Leu Ile
            260                 265                 270
Ile Ser Tyr Glu Thr Phe Arg Leu His Val Gly Val Leu Gln Lys Gly

        275                 280                 285
Ser Val Gly Leu Val Ile Cys Asp Glu Gly His Arg Leu Lys Asn Ser
    290                 295                 300
Glu Asn Gln Thr Tyr Gln Ala Leu Asp Ser Leu Asn Thr Ser Arg Arg
305                 310                 315                 320
Val Leu Ile Ser Gly Thr Pro Ile Gln Asn Asp Leu Leu Glu Tyr Phe
                325                 330                 335
Ser Leu Val His Phe Val Asn Ser Gly Ile Leu Gly Thr Ala His Glu
            340                 345                 350
Phe Lys Lys His Phe Glu Leu Pro Ile Leu Lys Gly Arg Asp Ala Ala
        355                 360                 365
Ala Ser Glu Ala Asp Arg Gln Leu Gly Glu Glu Arg Leu Arg Glu Leu
    370                 375                 380
Thr Ser Ile Val Asn Arg Cys Leu Ile Arg Arg Thr Ser Asp Ile Leu
385                 390                 395                 400
Ser Lys Tyr Leu Pro Val Lys Ile Glu Gln Val Val Cys Cys Arg Leu
                405                 410                 415
Thr Pro Leu Gln Thr Glu Leu Tyr Lys Arg Phe Leu Arg Gln Ala Lys
            420                 425                 430
Pro Ala Glu Glu Leu Leu Glu Gly Lys Met Ser Val Ser Ser Leu Ser
        435                 440                 445
Ser Ile Thr Ser Leu Lys Lys Leu Cys Asn His Pro Ala Leu Ile Tyr
    450                 455                 460
Asp Lys Cys Val Glu Glu Glu Asp Gly Phe Val Gly Ala Leu Asp Leu
465                 470                 475                 480
Phe Pro Pro Gly Tyr Ser Ser Lys Ala Leu Glu Pro Gln Leu Ser Gly
                485                 490                 495
Lys Met Leu Val Leu Asp Tyr Ile Leu Ala Val Thr Arg Ser Arg Ser
            500                 505                 510
Ser Asp Lys Val Val Leu Val Ser Asn Tyr Thr Gln Thr Leu Asp Leu
        515                 520                 525
Phe Glu Lys Leu Cys Arg Ala Arg Arg Tyr Leu Tyr Val Arg Leu Asp
    530                 535                 540
Gly Thr Met Ser Ile Lys Lys Arg Ala Lys Val Val Glu Arg Phe Asn
545                 550                 555                 560
Ser Pro Ser Ser Pro Asp Phe Val Phe Met Leu Ser Ser Lys Ala Gly
                565                 570                 575
Gly Cys Gly Leu Asn Leu Ile Gly Ala Asn Arg Leu Val Met Phe Asp
            580                 585                 590
Pro Asp Trp Asn Pro Ala Asn Asp Glu Gln Ala Met Ala Arg Val Trp
        595                 600                 605
Arg Asp Gly Gln Lys Lys Thr Cys Tyr Ile Tyr Arg Leu Leu Ser Ala
    610                 615                 620
Gly Thr Ile Glu Glu Lys Ile Phe Gln Arg Gln Ser His Lys Lys Ala
625                 630                 635                 640

Leu Ser Ser Cys Val Val Asp Glu Glu Gln Asp Val Glu Arg His Phe
                645                 650                 655
Ser Leu Gly Glu Leu Lys Glu Leu Phe Ile Leu Asp Glu Ala Ser Leu
            660                 665                 670
Ser Asp Thr His Asp Arg Leu His Cys Arg Arg Cys Val Asn Ser Arg
        675                 680                 685
Gln Ile Arg Pro Pro Pro Asp Gly Ser Asp Cys Thr Ser Asp Leu Ala
    690                 695                 700
Gly Trp Asn His Cys Thr Asp Lys Trp Gly Leu Arg Asp Glu Val Leu
705                 710                 715                 720
Gln Ala Ala Trp Asp Ala Ala Ser Thr Ala Ile Thr Phe Val Phe His
                725                 730                 735
Gln His Ser His Glu Glu Gln Arg Gly Leu Arg Ala Thr Gly
            740                 745                 750

(2) INFORMATION FOR SEQ ID NO:11:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: cDNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:11:

CTAATCTCTC GTCTCGGC                                                18

(2) INFORMATION FOR SEQ ID NO:12:

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: cDNA
(iii) HYPOTHETICAL: NO
(iv) ANTISENSE: NO
(v) FRAGMENT TYPE:

(vi) ORIGINAL SOURCE:

(xi) SEQUENCE DESCRIPTION: SEQ ID NO:12:

TGACCCAGGG CTATTCCCA 19

(2) INFORMATION FOR SEQ ID NO:13:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: cDNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:13:

TAGGCTGCAG GATCCTTG 18

(2) INFORMATION FOR SEQ ID NO:14:

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: cDNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:14:

CTAGAAACCA AATCCTGGC 19

(2) INFORMATION FOR SEQ ID NO:15:

(i) SEQUENCE CHARACTERISTICS:

(A) LENGTH: 19 base pairs
(B) TYPE: nucleic acid
(C) STRANDEDNESS: single
(D) TOPOLOGY: linear

(ii) MOLECULE TYPE: cDNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:15:

CCTGGCACTT AATAAGCAC 19

(2) INFORMATION FOR SEQ ID NO:16:

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: cDNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:16:

TGACTGGGCA CAGACATAC 19

(2) INFORMATION FOR SEQ ID NO:17:

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: cDNA
(iii) HYPOTHETICAL: NO
(iv) ANTISENSE: NO
(v) FRAGMENT TYPE:

(vi) ORIGINAL SOURCE:

(xi) SEQUENCE DESCRIPTION: SEQ ID NO:17:

CCATAACATC TCCAGTCAG 19

(2) INFORMATION FOR SEQ ID NO:18:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: cDNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:18:

CAGGCACACG TACATATG 18

(2) INFORMATION FOR SEQ ID NO:19:

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: cDNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:19:

CTGGAGCTCC TAAACATAG 19

(2) INFORMATION FOR SEQ ID NO:20:

(i) SEQUENCE CHARACTERISTICS:

(A) LENGTH: 19 base pairs
(B) TYPE: nucleic acid
(C) STRANDEDNESS: single
(D) TOPOLOGY: linear

(ii) MOLECULE TYPE: cDNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:20:

GCGTGCATAT ACAGAGAAC 19

(2) INFORMATION FOR SEQ ID NO:21:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: cDNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:21:

TTGCCCATGT GTGAGCAC 18

(2) INFORMATION FOR SEQ ID NO:22:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: cDNA
(iii) HYPOTHETICAL: NO
(iv) ANTISENSE: NO
(v) FRAGMENT TYPE:

(vi) ORIGINAL SOURCE:

(xi) SEQUENCE DESCRIPTION: SEQ ID NO:22:

ACTTTGGCAC CTACGCTG 18

(2) INFORMATION FOR SEQ ID NO:23:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: cDNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:23:

AGCGTAGGTG CCAAAGTG 18

(2) INFORMATION FOR SEQ ID NO:24:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: cDNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:24:

AGTTCTTCAC CAGGCTGG 18

(2) INFORMATION FOR SEQ ID NO:25:

(i) SEQUENCE CHARACTERISTICS:

(A) LENGTH: 19 base pairs
(B) TYPE: nucleic acid
(C) STRANDEDNESS: single
(D) TOPOLOGY: linear

(ii) MOLECULE TYPE: cDNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:25:

CTGCAGTGCA TCACATTGA 19

(2) INFORMATION FOR SEQ ID NO:26:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: cDNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:26:

TAAGAAAGCA GCAGGCTG 18

(2) INFORMATION FOR SEQ ID NO:27:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: cDNA
(iii) HYPOTHETICAL: NO
(iv) ANTISENSE: NO
(v) FRAGMENT TYPE:

(vi) ORIGINAL SOURCE:

(xi) SEQUENCE DESCRIPTION: SEQ ID NO:27:

AGATTCTGAA TTGTTCCC 18

(2) INFORMATION FOR SEQ ID NO:28:

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: cDNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:28:

GGCAATACTC AGTGAAGAG 19

(2) INFORMATION FOR SEQ ID NO:29:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: cDNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:29:

TACCGTATAG GGAATGCC 18

(2) INFORMATION FOR SEQ ID NO:30:

(i) SEQUENCE CHARACTERISTICS:

(A) LENGTH: 18 base pairs
(B) TYPE: nucleic acid
(C) STRANDEDNESS: single
(D) TOPOLOGY: linear

(ii) MOLECULE TYPE: cDNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:30:

AAAGACAAGG CAGGGCTC 18

(2) INFORMATION FOR SEQ ID NO:31:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: cDNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:31:

CTGCCATCAC TAGCTGTG 18

(2) INFORMATION FOR SEQ ID NO:32:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: cDNA
(iii) HYPOTHETICAL: NO
(iv) ANTISENSE: NO
(v) FRAGMENT TYPE:

(vi) ORIGINAL SOURCE:

(xi) SEQUENCE DESCRIPTION: SEQ ID NO:32:

AAGGATTGGC CATGGATG 18

(2) INFORMATION FOR SEQ ID NO:33:

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: cDNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:33:

GCATGGCAAT TTTACCAGC 19

(2) INFORMATION FOR SEQ ID NO:34:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: cDNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:34:

TTCAGGAGCT AGGCTTTG 18

(2) INFORMATION FOR SEQ ID NO:35:

(i) SEQUENCE CHARACTERISTICS:

(A) LENGTH: 19 base pairs
(B) TYPE: nucleic acid
(C) STRANDEDNESS: single
(D) TOPOLOGY: linear

(ii) MOLECULE TYPE: cDNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:35:

ATCAAGGTGT TCTCAGAGG 19

(2) INFORMATION FOR SEQ ID NO:36:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: cDNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:36:

TTGCTCACTC TCACAGAG 18

(2) INFORMATION FOR SEQ ID NO:37:

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: cDNA
(iii) HYPOTHETICAL: NO
(iv) ANTISENSE: NO
(v) FRAGMENT TYPE:

(vi) ORIGINAL SOURCE:

(xi) SEQUENCE DESCRIPTION: SEQ ID NO:37:

CTAGTGAACA CTGAAGTGG 19

(2) INFORMATION FOR SEQ ID NO:38:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: cDNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:38:

GAAACAGGAC AGCCCTAG 18

(2) INFORMATION FOR SEQ ID NO:39:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: cDNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:39:

AAGAAGCCTG GGCCATTG 18

(2) INFORMATION FOR SEQ ID NO:40:

(i) SEQUENCE CHARACTERISTICS:

(A) LENGTH: 18 base pairs
(B) TYPE: nucleic acid
(C) STRANDEDNESS: single
(D) TOPOLOGY: linear

(ii) MOLECULE TYPE: cDNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:40:

AGACAGACAG TAGGGGAG 18

(2) INFORMATION FOR SEQ ID NO:41:

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: cDNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:41:

TCCCCCTAAT CATTGAAGC 19

(2) INFORMATION FOR SEQ ID NO:42:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: cDNA
(iii) HYPOTHETICAL: NO
(iv) ANTISENSE: NO
(v) FRAGMENT TYPE:

(vi) ORIGINAL SOURCE:

(xi) SEQUENCE DESCRIPTION: SEQ ID NO:42:

CTGATGATAC TGCATTGG 18

(2) INFORMATION FOR SEQ ID NO:43:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: cDNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:43:

CAGGATCCCA GTTTAGGC 18

(2) INFORMATION FOR SEQ ID NO:44:

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: cDNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:44:

CTGAAGATCT CGTTCCATG 19

(2) INFORMATION FOR SEQ ID NO:45:

(i) SEQUENCE CHARACTERISTICS:

(A) LENGTH: 18 base pairs
(B) TYPE: nucleic acid
(C) STRANDEDNESS: single
(D) TOPOLOGY: linear

(ii) MOLECULE TYPE: cDNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:45:

ATGGCTAAGC GCTGTATC 18

(2) INFORMATION FOR SEQ ID NO:46:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: cDNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:46:

ACTGTGTGGG TAGCTTAG 18

(2) INFORMATION FOR SEQ ID NO:47:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: cDNA
(iii) HYPOTHETICAL: NO
(iv) ANTISENSE: NO

(v) FRAGMENT TYPE:
(vi) ORIGINAL SOURCE:

(xi) SEQUENCE DESCRIPTION: SEQ ID NO:47:

AGTGCCCTAA CCATTATC 18

(2) INFORMATION FOR SEQ ID NO:48:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: cDNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:48:

TGGAAGACGA AGGTGATA 18

(2) INFORMATION FOR SEQ ID NO:49:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: cDNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:49:

CCACTGCACT GATAAGTG 18

(2) INFORMATION FOR SEQ ID NO:50:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: cDNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:50:

ATGCAGCCCT TCTTGATG 18

(2) INFORMATION FOR SEQ ID NO:51:

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: cDNA
(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:51:

AGCCCTGACT TTGTCTTCA 19

(2) INFORMATION FOR SEQ ID NO:52:

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: cDNA

(iii) HYPOTHETICAL: NO
(iv) ANTISENSE: NO
(v) FRAGMENT TYPE:
(vi) ORIGINAL SOURCE:

(xi) SEQUENCE DESCRIPTION: SEQ ID NO:52:

GCTTGTTCAT CCATTGGCT 19